(19) Europäisches Patentamt
European Patent Office
Office européen des brevets

(11) EP 1 867 634 B1

(12) **EUROPEAN PATENT SPECIFICATION**

(45) Date of publication and mention of the grant of the patent:
**26.05.2010 Bulletin 2010/21**

(21) Application number: **06731365.0**

(22) Date of filing: **31.03.2006**

(51) Int Cl.:
***C07D 211/14*** *(2006.01)* ***A61K 51/00*** *(2006.01)*
***C07F 7/22*** *(2006.01)*

(86) International application number:
**PCT/JP2006/307418**

(87) International publication number:
**WO 2006/107106 (12.10.2006 Gazette 2006/41)**

(54) **LIGAND FOR VESICULAR ACETYLCHOLINE TRANSPORTER**

LIGAND FÜR DEN VESIKULÄREN ACETYLCHOLINTRANSPORTER

LIGAND POUR LE TRANSPORTEUR D ACETYLCHOLINE VESICULAIRE

(84) Designated Contracting States:
**DE GB**

(30) Priority: **01.04.2005 JP 2005106824**

(43) Date of publication of application:
**19.12.2007 Bulletin 2007/51**

(73) Proprietor: **National University Corporation Kanazawa University**
**Kanazawa-shi,**
**Ishikawa 920-1164 (JP)**

(72) Inventors:
- **ISHIWATA, Kiichi**
  **Tokyo Metrop. Inst. of Gerontology, 35-2**
  **Sakae-cho**
  **Itabashi-ku**
  **Tokyo 173-0015 (JP)**
- **SHIBA, Kazuhiro,**
  **Kanazawa University**
  **Kanazawa-shi, Ishikawa 9201164 (JP)**
- **TSUKADA, Hideo**
  **Hamamatsu-shi, Shizuoka 4358558 (JP)**
- **NISHIYAMA, Shingo**
  **Hamamatsu-shi, Shizuoka 4358558 (JP)**

(74) Representative: **von Kreisler Selting Werner**
**Deichmannhaus am Dom**
**Bahnhofsvorplatz 1**
**50667 Köln (DE)**

(56) References cited:

**WO-A1-01/07427 JP-A- 07 507 547**
**US-A- 5 338 852**

- **KAZUHIRO SHIBA, KAZUMA OGAWA, KIICHI ISHIWATA, KAZUYOSHI YAJIMA, HIROFUMI MORI: "Synthesis and binding affinities of methylvesamicol analogs for the acetylcholine transporter and sigma receptor" BIOORGANIC & MEDICINAL CHEMISTRY LETTERS, vol. 14, 24 January 2006 (2006-01-24), pages 2620-2626, XP002513187**
- **MICHAEL R. KILBOURN, YONG-WOON JUNG, MICHAEL S. HAKA, DAVID L. GILDERSLEEVE, DAVID E. KUHL, DONALD M. WIELAND: "Mouse brain distribution of a carbon-11 labeled vesamicol derivative: Presynaptic marker of cholinergic neurons" LIFE SCIENCES, vol. 47, no. 21, 1990, pages 1955-1963, XP002513200**
- **ROGERS G.A. ET AL.: 'Synthesis, in Vitro Acetylcholine-Storage-Blocking Activities, and Biological Properties of Derivatives and Analogues of trans-2-(4-Phenylpiperidino) cyclohexanol (Versamicol)' JOURNAL OF MEDICINAL CHEMISTRY vol. 32, no. 6, 01 June 1989, pages 1217 - 1230, XP000371825**
- **SHIBA K. ET AL.: 'Evaluation of radioiodinated (-)-o-iodovesamicol as a radiotracer for mapping the vesicular acetylcholine transporter' ANNALS OF NUCLEAR MEDICINE vol. 17, no. 6, 2003, pages 451 - 456, XP003001637**
- **SHIBA K. ET AL.: 'Characterization of radioiodinated (-)-ortho-iodovesamicol binding in rat brain preparations' LIFE SCIENCES vol. 71, no. 13, 2002, pages 1591 - 1598, XP003001638**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to a compound (ligand) having specific binding affinity for a vesicular acetylcholine transporter (hereinafter to be abbreviated as VAChT).

### Background Art

2-(4-Phenylpiperidino)-cyclohexanol (vesamicol) is known to have strong binding affinity for VAChT in the presynaptic cholinergic neuron. Hence, many vesamicol analogues have been developed as diagnostic reagents for diagnosing cholinergic neurodegenerative disorders (for example, Alzheimer's disease). For example, a vesamicol analogue incorporating iodine at the meta-position of the benzene ring moiety of vesamicol ((-)-m-iodovesamicol) has been reported to be useful as a VAChT mapping agent. However, vesamicol and some of the analogues thereof have been reported to also bind to heterologous receptors, including sigma receptors (σ-1 and σ-2), as well as to VAChT.
Shiba et al. examined enantiomers of vesamicol analogues incorporating iodine at the ortho-, meta-, or para-position of the benzene ring moiety of vesamicol for affinity for VAChT and sigma receptors in vitro. As a result, they showed that (-)-o-iodovesamicol, like (-)-vesamicol, have higher binding affinity for VAChT and lower binding affinity for sigma receptors compared to other analogues (see, for example, Shiba et al., Life Sciences 71 (2002) 1591-1598).
Moreover, Shiba et al. evaluated the ability of (-)-[$^{125}$I]-o-iodovesamicol as a radiotracer for mapping VAChT. As a result, (-)-o-iodovesamicol clarified to be useful as a VAChT mapping agent (for example, see Shiba et al., Annals of Nuclear Medicine Vol. 17, No. 6, 451-456, 2003).

### Disclosure of the Invention

In utilizing (-)-o-iodovesamicol in human patients, (-)-[$^{123}$I]-o-iodovesamicol, which has a shorter half-life than the above-described (-)-[$^{125}$I]-o-iodovesamicol, can be expected as a VAChT mapping agent using single photon emission computed tomography (hereinafter abbreviated SPECT). However, SPECT cannot be said to be significantly better other imaging methods in terms of sensitivity, resolution, and quantitative analyzability, though it is better in terms of versatility. For example, by SPECT, it is difficult to detect slight neurochemical changes in the brain in early neurodegenerative disorders.
Therefore, the present inventors took note of mapping VAChT using positron emission tomography (hereinafter abbreviated as PET) superior to SPECT in the sensitivity, resolution and quantification.
From the foregoing, the present invention aims at providing a novel compound useful as a reagent (radiotracer) for mapping VAChT using PET superior to SPECT in the sensitivity, resolution and quantification and a production method thereof, as well as a diagnostic reagent comprising the above-mentioned novel compound, which is used for diagnosing cholinergic neurodegenerative disorders (e.g., Alzheimer's disease, memory disorder, learning disorder, schizophrenia, cognitive dysfunction, hyperactivity disorder, anxiety neurosis, depression, analgia, Parkinson's disease and the like) and the like, characterized by a decrease in VAChT.
The present inventors have conducted intensive studies in an attempt to solve the above-mentioned problems and found that a novel vesamicol analog (i.e., (-)-o-methylvesamicol) wherein a methyl group is introduced into the ortho position of the benzene ring moiety of (-)-vesamicol has high binding affinity for VAChT and low binding affinity for σ receptor, which are comparable to those of (-)-vesamicol. They have got a conception of utilizing a novel compound (i.e., (-)-[$^{11}$C]-o-methylvesamicol) wherein $^{11}CH_3$ group, which is a $CH_3$ group labeled with $^{11}$C, a positron nuclide, has been introduced as a methyl group moiety of (-)-o-methylvesamicol, as a diagnostic reagent for PET, and further conducted investigation, which resulted in the completion of the present invention.
Accordingly, the present invention relates to the following.

(1) A compound represented by the following formula (I):

(-) 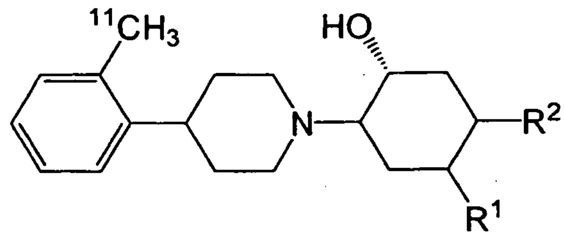 (I)

wherein $R^1$ and $R^2$ are the same or different and each is a hydrogen atom, a halogen atom, a hydroxyl group, an amino group, a $C_{1-6}$ acyl group, a carboxy group, a $C_{1-6}$ alkoxy group, a $C_{1-6}$ alkyl group or a phenyl group, or $R^1$ and $R^2$ in combination optionally form a ring together with the adjacent carbon atom (the ring optionally has one or more substituents selected from the group consisting of a halogen atom, a hydroxyl group, an amino group, a $C_{1-6}$ acyl group, a carboxy group, a $C_{1-6}$ alkoxy group, a $C_{1-6}$ alkyl group), or a salt thereof.

(2) The compound of the above-mentioned (1), wherein $R^1$ and $R^2$ are hydrogen atoms (i.e., (-)-[$^{11}$C]-o-methylvesamicol), or a salt thereof.

(3) A method of producing a compound represented by the following formula (I):

(-) 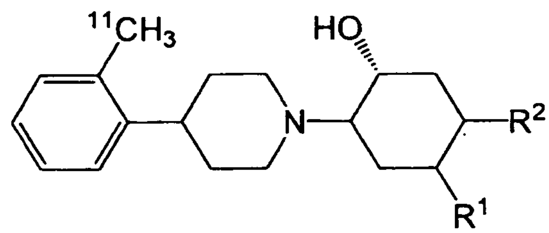 (I)

wherein $R^1$ and $R^2$ are the same or different and each is a hydrogen atom, a halogen atom, a hydroxyl group, an amino group, a $C_{1-6}$ acyl group, a carboxy group, a $C_{1-6}$ alkoxy group, a $C_{1-6}$ alkyl group or a phenyl group, or $R^1$ and $R^2$ in combination optionally form a ring together with the adjacent carbon atom (the ring optionally has one or more substituents selected from the group consisting of a halogen atom, a hydroxyl group, an amino group, a $C_{1-6}$ acyl group, a carboxy group, a $C_{1-6}$ alkoxy group, a $C_{1-6}$ alkyl group), or a salt thereof, which comprises a step of reacting a compound of the following formula (II):

(-) 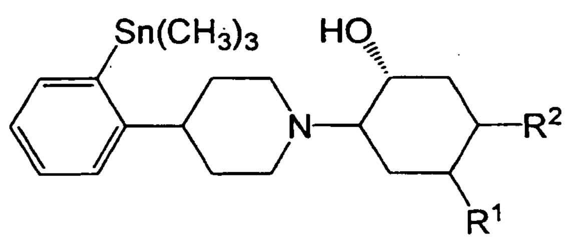 (II)

wherein $R^1$ and $R^2$ are as defined for the aforementioned formula (I),
or a salt thereof with $^{11}CH_3I$.

(4) The production method of the above-mentioned (3), wherein $R^1$ and $R^2$ in the aforementioned formula are hydrogen atoms.

(5) A diagnostic reagent for a cholinergic neurodegenerative disorder, which comprises a compound of the above-mentioned (1) or (2), or a salt thereof.

(6) The diagnostic reagent of the above-mentioned (5), wherein the aforementioned cholinergic neurodegenerative disorder is Alzheimer's disease, memory disorder, learning disorder, schizophrenia, cognitive dysfunction, hyperactivity disorder, anxiety neurosis, depression, analgia or Parkinson's disease.

(7) A compound represented by the following formula (II):

(-) 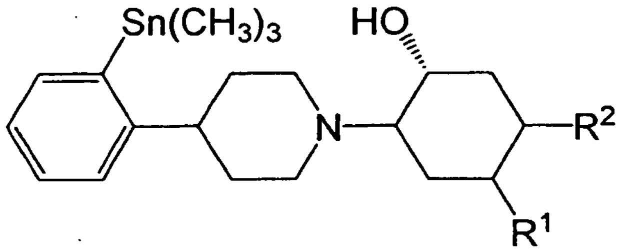 (II)

wherein $R^1$ and $R^2$ are the same or different and each is a hydrogen atom, a halogen atom, a hydroxyl group, an amino group, a $C_{1-6}$ acyl group, a carboxy group, a $C_{1-6}$ alkoxy group, a $C_{1-6}$ alkyl group or a phenyl group, or $R^1$ and $R^2$ in combination optionally form a ring together with the adjacent carbon atom (the ring optionally has one or more substituents selected from the group consisting of a halogen atom, a hydroxyl group, an amino group, a $C_{1-6}$ acyl group, a carboxy group, a $C_{1-6}$ alkoxy group, a $C_{1-6}$ alkyl group), or a salt thereof.

(8) The compound of the above-mentioned (7) or a salt thereof, wherein $R^1$ and $R^2$ in the aforementioned formula are hydrogen atoms.

(9) A compound represented by the following formula (Ia):

(-) 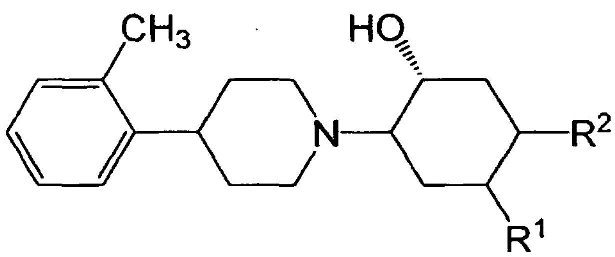 (Ia)

wherein $R^1$ and $R^2$ are the same or different and each is a hydrogen atom, a halogen atom, a hydroxyl group, an amino group, a $C_{1-6}$ acyl group, a carboxy group, a $C_{1-6}$ alkoxy group, a $C_{1-6}$ alkyl group or a phenyl group, or $R^1$ and $R^2$ in combination optionally form a ring together with the adjacent carbon atom (the ring optionally has one or more substituents selected from the group consisting of a halogen atom, a hydroxyl group, an amino group, a $C_{1-6}$ acyl group, a carboxy group, a $C_{1-6}$ alkoxy group, a $C_{1-6}$ alkyl group), or a salt thereof.

(10) The compound of the above-mentioned (9), wherein $R^1$ and $R^2$ in the aforementioned formula are hydrogen atoms (i.e., (-)-o-methylvesamicol), or a salt thereof.

(11) A compound represented by the following formula (I):

(-) 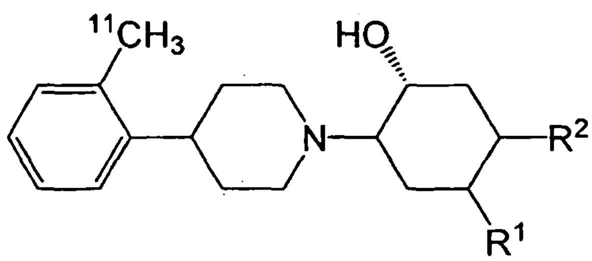 (I)

wherein $R^1$ and $R^2$ are the same or different and each is a hydrogen atom, a halogen atom, a hydroxyl group, an amino group, a $C_{1-6}$ acyl group, a carboxy group, a $C_{1-6}$ alkoxy group, a $C_{1-6}$ alkyl group or a phenyl group, or $R^1$ and $R^2$ in combination optionally form a ring together with the adjacent carbon atom (the ring optionally has one or more substituents selected from the group consisting of a halogen atom, a hydroxyl group, an amino group, a $C_{1-6}$ acyl group, a carboxy group, a $C_{1-6}$ alkoxy group, a $C_{1-6}$ alkyl group),
or a salt thereof, which is a diagnostic reagent for a cholinergic neurodegenerative disorder.

(12) The compound of the above-mentioned (11), wherein $R^1$ and $R^2$ in the aforementioned formula are hydrogen atoms (i.e., (-)-[$^{11}$C]-o-methylvesamicol), or a salt thereof.

(13) The compound of the above-mentioned (11) or (12), or a salt thereof, wherein the aforementioned cholinergic

neurodegenerative disorder is Alzheimer's disease, memory disorder, learning disorder, schizophrenia, cognitive dysfunction, hyperactivity disorder, anxiety neurosis, depression, analgia or Parkinson's disease.

(14) A method of diagnostically imaging a cholinergic neurodegenerative disorder, which comprises administering, to a subject animal with a cholinergic neurodegenerative disorder, a compound represented by the following formula (I):

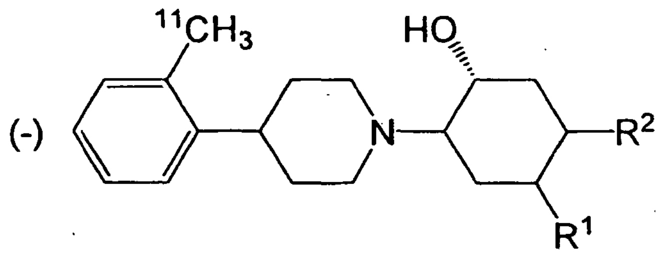

(I)

wherein $R^1$ and $R^2$ are the same or different and each is a hydrogen atom, a halogen atom, a hydroxyl group, an amino group, a $C_{1-6}$ acyl group, a carboxy group, a $C_{1-6}$ alkoxy group, a $C_{1-6}$ alkyl group or a phenyl group, or $R^1$ and $R^2$ in combination optionally form a ring together with the adjacent carbon atom (the ring optionally has one or more substituents selected from the group consisting of a halogen atom, a hydroxyl group, an amino group, a $C_{1-6}$ acyl group, a carboxy group, a $C_{1-6}$ alkoxy group, a $C_{1-6}$ alkyl group),

or a salt thereof, and photographing an image of a cholinergic neurodegenerative disorder part.

(15) The diagnostic imaging method of the above-mentioned (14), wherein $R^1$ and $R^2$ in the aforementioned formula are hydrogen atoms.

(16) The diagnostic imaging method of the above-mentioned (14) or (15), wherein the aforementioned cholinergic neurodegenerative disorder is Alzheimer's disease, memory disorder, learning disorder, schizophrenia, cognitive dysfunction, hyperactivity disorder, anxiety neurosis, depression, analgia or Parkinson's disease.

**Brief Description of the Drawings**

Figure 1 shows a schematic diagram of the apparatus used to synthesize the (-)-[$^{11}$C]-o-methylvesamicol of the present invention in Example 4. In Figure 1, TG indicates a target (wherein [$^{11}$C]$CO_2$ is produced by $^{14}N(P,\alpha)^{11}C$ nuclear reaction), MEI indicates a [$^{11}$C] methyl iodide synthesizer, VA1 indicates a syringe for injection of the reaction reagent, VA2 indicates a syringe for injection of the HPLC eluent, and RV1 indicates a reaction vessel.

Figure 2 shows photographs showing the results of ex vivo autoradiography of the rat brain using the (-)-[$^{11}$C]-o-methylvesamicol of the present invention in Example 7. The upper panel of Figure 2 shows photographs of four sections of the brain of a rat killed 15 minutes after injection of (-)-[$^{11}$C]-o-methylvesamicol; the lower panel of Figure 2 shows photographs of four sections of the brain of a rat killed 30 minutes after injection of (-)-[$^{11}$C]-o-methylvesamicol.

Figure 3 shows schematic diagrams of the photographs in the upper panel of Figure 2, indicating various parts of the rat brain by the arrows.

Figure 4 shows PET image photographs of conscious monkey brains, obtained by picturizing the image data obtained at 15 minutes to 60 minutes after administration of the (-)-[$^{11}$C]-o-methylvesamicol of the present invention in Example 8. The left panel is a brain image from a healthy monkey after administration of (-)-[$^{11}$C]-o-methylvesamicol; the right panel is a brain image from a neurolysis monkey after administration of (-)-[$^{11}$C]-o-methylvesamicol. In each photograph, the left hemisphere of the brain is on the left, and the right hemisphere of the brain is on the right. Cerebral cortex is indicated by the arrows. The image photographs are originally color photographs; the red region is a region wherein much (-)-[$^{11}$C]-o-methylvesamicol accumulates, that is, a region of high VAChT density. In the right hemisphere of the brain (neurolysis side) of the model monkey on the right panel (neurolysis monkey), VAChT density has decreased due to neurolysis, and the accumulation of (-)-[$^{11}$C]-o-methylvesamicol is low, resulting in the green color showing that the VAChT density is low in the region. For example, in the case of Alzheimer's disease as well, because it is considered that the VAChT density has decreased, an affected lesion appears as a defective image like in green color, and (-)-[$^{11}$C]-o-methylvesamicol can be utilized for the diagnosis.

Figure 5 shows diagrammatic illustrations of the photographs in Figure 4, prepared to clarify the red regions in Figure 4, with the red regions shown in solid black.

## Detailed Description of the Invention

The present invention provides a compound represented by the following formula (I):

(-) 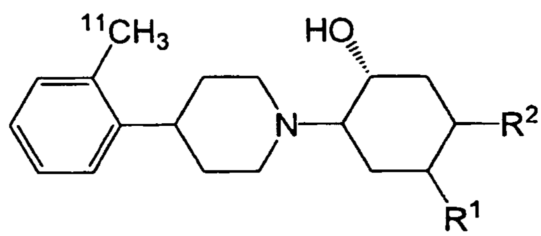 (I)

wherein $R^1$ and $R^2$ are as defined above, (hereinafter to be referred to as compound (I)), or a salt thereof, which has specific binding affinity for VAChT and is labeled with a positron nuclide $^{11}C$.

In the above-mentioned formula (I), and the below-mentioned formula (II) and the formula (Ia), $R^1$ and $R^2$ are the same or different and each is a hydrogen atom, a halogen atom, a hydroxyl group, an amino group, a $C_{1-6}$ acyl group, a carboxy group, a $C_{1-6}$ alkoxy group, a $C_{1-6}$ alkyl group or a phenyl group, where $R^1$ and $R^2$ are preferably hydrogen atoms.

As the halogen atom, for example, fluorine, chlorine, bromine, iodine and the like can be mentioned.

As the $C_{1-6}$ acyl group, for example, formyl, $C_{1-5}$ alkylcarbonyl group such as acetyl, propionyl, butyryl, isobutyryl, pivaloyl, valeryl, isovaleryl, hexanoyl and the like, and the like can be mentioned.

As the $C_{1-6}$ alkoxy group, alkoxy group having 1 to 6 carbon atoms such as methoxy, ethoxy, propoxy, isopropoxy, butoxy, isobutoxy, sec-butoxy, pentyloxy, hexyloxy and the like can be mentioned.

As the $C_{1-6}$ alkyl group, alkyl group having 1 to 6 carbon atoms such as methyl, ethyl, propyl, isopropyl, butyl, isobutyl, sec-butyl, tert-butyl, pentyl, hexyl and the like can be mentioned.

Alternatively, in the above-mentioned formula (I), the below-mentioned formula (II) and the formula (Ia), $R^1$ and $R^2$ in combination optionally form a ring together with the adjacent carbon atom. As such ring, for example, cyclopentyl, cyclohexyl, benzene ring, 5- or 6-membered aromatic heterocycle and the like can be mentioned.

These rings optionally have one or more substituents selected from the group consisting of a halogen atom, a hydroxyl group, an amino group, a $C_{1-6}$ acyl group, a carboxy group, a $C_{1-6}$ alkoxy group and a $C_{1-6}$ alkyl group. Here, halogen atom, $C_{1-6}$ acyl group, $C_{1-6}$ alkoxy group and $C_{1-6}$ alkyl group are as defined above.

As the salt of compound (I), a pharmaceutically acceptable salt is preferable and, for example, salt with inorganic base, salt with organic base, salt with inorganic acid, salt with organic acid, salt with basic or acidic amino acid and the like can be mentioned.

As the inorganic base that forms a salt, for example, alkali metal such as sodium, potassium and the like; alkaline earth metal such as calcium, magnesium and the like; aluminum, ammonium and the like can be mentioned. As the organic base that forms a salt, for example, trimethylamine, triethylamine, pyridine, picoline, ethanolamine, diethanolamine, triethanolamine, dicyclohexylamine, N',N'-dibenzylethylenediamine and the like can be mentioned. As the inorganic acid that forms a salt, for example, hydrochloric acid, hydrobromide acid, nitric acid, sulfuric acid, phosphoric acid and the like can be mentioned. As the organic acid that forms a salt, for example, formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like can be mentioned. As the basic amino acid that forms a salt, for example, arginine, lysin, ornithine and the like can be mentioned, and as the acidic amino acid that forms a salt, for example, aspartic acid, glutamic acid and the like can be mentioned.

Compound (I) can be converted to a pharmaceutically acceptable salt thereof by a method known in the art.

Compound (I) and a salt thereof can be produced by appropriately applying the following Production Method 1, or an organic chemical synthesis method known in the art.

(Production Method 1)

(Scheme 1-1)

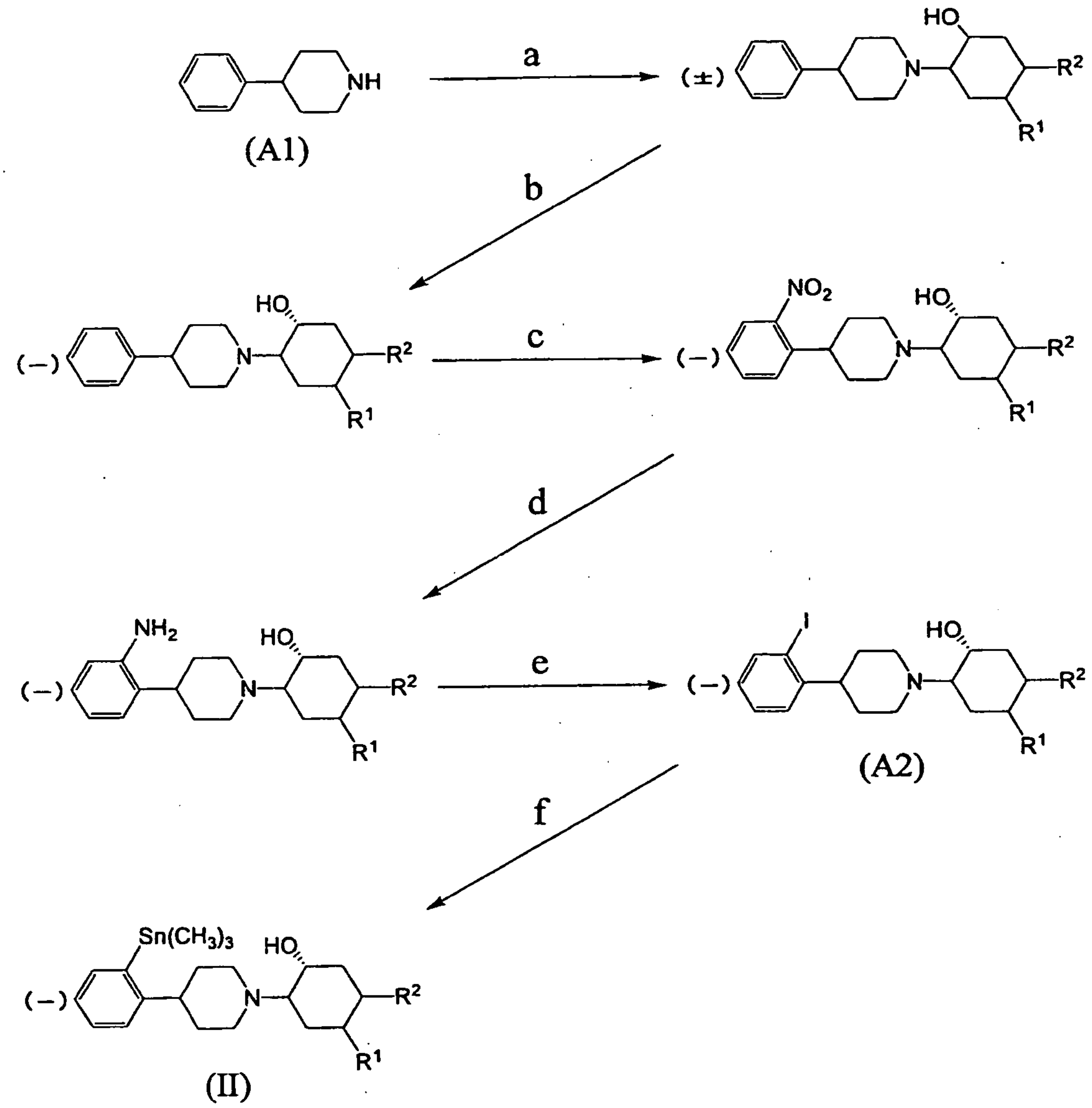


The reagents and the like used in each step of Scheme 1-1 are shown below.

Step a:

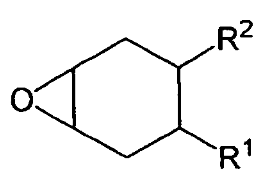

Step b: (-)-di-p-toluoyl-L-tartaric acid;
Step c: $HNO_3$, $H_2SO_4$;
Step d: Fe, HCl;
Step e: $NaNO_2$, HCl, KI;
Step f: $[(CH_3)_3Sn]_2$, catalyst

In each formula, $R^1$ and $R^2$ are as defined above.

In Scheme 1-1, a compound represented by the formula (A2) (hereinafter to be referred to as compound (A2))

can be synthesized from 4-phenylpiperidine (compound represented by the formula (A1)), for example, according to the method described in Shiba et al., Life Sciences 71 (2002) 1591-1598 and Shiba et al., Annals of Nuclear Medicine Vol. 17, No.6, 451-456, 2003, via steps a - e.

In Scheme 1-1, a compound represented by the formula (II) (hereinafter to be referred to as compound (II)) can be synthesized from compound (A2) by a halogen-metal exchange stannylation reaction. This reaction is generally performed by stirring compound (A2) and hexamethyl di-tin in an anhydride solvent under an inert gas (for example, nitrogen, argon and the like) stream in the presence of a catalyst generally for about 1 - 10 min, preferably, about 3 - 5 min, and then reacting at any appropriate temperature between -15˚C to 200˚C according to the solvent to be used for about 15 - 20 hr.

The solvent to be used for this reaction is not particularly limited as long as it does not inhibit the reaction and, for example, toluene, tetrahydrofuran (THF), triethylamine, hexane, a mixed solvent thereof and the like can be mentioned. As preferable solvent, toluene can be mentioned.

The amount of the solvent to be used for this reaction is generally about 20- to 50-fold weight, preferably, about 35-to 45-fold weight, relative to compound (A2).

As the catalyst to be used for this reaction, for example, tetrakis(tri-phenylphosphine)palladium(0)($(Ph_3P)_4Pd$ (0)), tris(di-benzylideneacetone)dipalladium($Pd_2(dba)_3$), tri-o-toluylphosphine($(o\text{-}Tol)_3P$) and the like can be mentioned, but the catalyst is not limited to these. These catalysts may be use in combination of two kinds or more. As preferable catalyst, tetrakis(tri phenylphosphine)palladium(0) can be mentioned.

The amount of the catalyst to be used for this reaction is generally about 0.01- to 10-fold mole, preferably, about 0.05- to 0.1-fold mole, relative to compound (A2).

(Scheme 1-2)

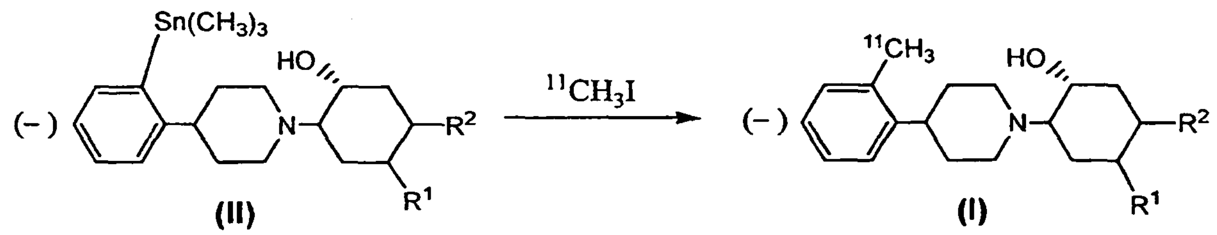


(II) (I)

In each formula, $R^1$ and $R^2$ are as defined above.

Compound (I) shown in Scheme 1-2 is generally synthesized by reacting compound (II) with [$^{11}$C]methyl iodide in a solvent in the presence of a catalyst.

The solvent to be used for this reaction is not particularly limited as long as it does not inhibit the reaction and, for example, N,N-dimethylformamide (DMF), dimethyl sulfoxide (DMSO), acetonitrile, 1,4-dioxane, xylene, 1,2-dimethoxyethane, dichloromethane, a mixed solvent thereof and the like can be mentioned. As preferable solvent, DMF can be mentioned.

The amount of the solvent to be used for this reaction is generally about 250- to 1000-fold weight, preferably, about 400- to 600-fold weight, relative to compound (II).

As the catalyst to be used for this reaction, for example, tris(di-benzylideneacetone)dipalladium($Pd_2(dba)_3$), tri-o-toluylphosphine($(o\text{-}Tol)_3P$), tetrakis(triphenylphosphine)palladium($Pd(PPh_3)_4$), tri-o-toluylphosphinepalladium($Pd[P(o\text{-}Tol)_3]_2$), copper(I) chloride(CuCl), potassium carbonate($K_2CO_3$) and the like can be mentioned, but the catalyst is not limited to these.

As preferable catalyst, a combination of tris(dibenzylideneacetone)dipalladium, tri-o-toluylphosphine, copper (I) chloride (CuCl) and potassium carbonate ($K_2CO_3$) can be mentioned.

The amount of the catalyst to be used for this reaction is generally about 0.1- to 150-fold mole, preferably about 1-to 100-fold mole, relative to compound (II).

The amount of [$^{11}$C]methyl iodide to be used for this reaction is 0.5 GBq - 100 GBq, preferably 10 GBq - 50 GBq, as the amount of radioactivity.

The temperature of methylation reaction is generally about 40˚C - 90˚C, preferably about 70˚C - 85˚C. The reaction time is generally about 3 - 10 min.

Since compound (I) or a salt thereof has specific binding affinity for VAChT and is labeled with $^{11}$C, which is a detectable positron nuclide in PET, it is useful as a reagent (radiotracer) for mapping VAChT using PET in human or non-human mammals (monkey, horse, bovine, swine, goat, sheep, dog, cat, guinea pig, rat, mouse, hamster and the like).

Compound (I) or a salt thereof is useful as a diagnostic reagent of cholinergic neurodegenerative disorders

characterized by a decrease in VAChT in human or non-human mammals (monkey, horse, bovine, swine, goat, sheep, dog, cat, guinea pig, rat, mouse, hamster and the like).

As the cholinergic neurodegenerative disorders, diseases characterized by a decrease in VAChT, such as Alzheimer's disease, memory disorder, learning disorder, schizophrenia, cognitive dysfunction, hyperactivity disorder, anxiety neurosis, depression, analgia, Parkinson's disease and the like.

When compound (I) or a salt thereof is used as a diagnostic reagent, compound (I) or a salt thereof in an amount effective for diagnosis is administered to the subject, preferably intravenously administered. The amount effective for diagnosis means an amount sufficient to enable in vivo localization of label and detection thereof.

When compound (I) or a salt thereof is used as an injection for intravenous administration, compound (I) or a salt thereof may be formulated as an aqueous injection together with a stabilizer (e.g., ascorbic acid), a solubilizer (e.g., β-cyclodextrins and the like), a dispersing agent (e.g., Tween 80, carboxymethylcellulose, sodium alginate and the like), a preservative (e.g., methylparaben, propylparaben, benzyl alcohol, chlorobutanol and the like), an isotonicity agent (e.g., sodium chloride, glycerol, sorbitol, glucose and the like) and the like, according to a conventional method, or dissolved, suspended or emulsified in vegetable oil (e.g., olive oil, sesame oil, peanut oil, cottonseed oil, corn oil and the like), propylene glycol and the like to give an oily injection.

The unit dose to be administered to an adult is generally an amount affording about 300 MBq - about 800 MBq, preferably about 400 MBq - 600 MBq, of radioactivity. The amount of the solution to be injected is about 10 ml - about 20 ml.

VAChT in the subject can be imaged by dynamic measurement by PET at 30 min - 90 min after intravenous administration of the diagnostic reagent of the present invention to the subject.

Since the positron nuclide $^{11}C$ used in the present invention has a very short half-life, compound (I) may be produced by [$^{11}C$] methylation of compound (II) immediately before analysis or diagnosis by PET at the actual site of analysis or diagnose, and used.

Accordingly, the present invention also provides a compound represented by the following formula (II):

(-) 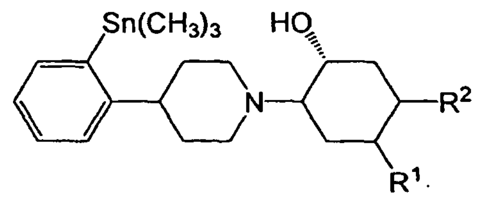 (II)

wherein $R^1$ and $R^2$ are as defined above (compound (II), or a salt thereof as a precursor of compound (I) ([$^{11}C$]-labeled vesamicol).

As the salt of compound (II), the salts exemplified for the aforementioned compound (I) can be mentioned. Compound (II) can be converted to a pharmaceutically acceptable salt thereof by a method known in the art.

The present invention also provides a compound represented by the following formula (Ia):

(-) 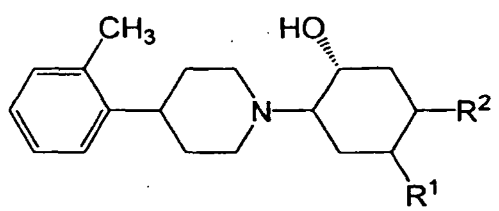 (Ia)

wherein $R^1$ and $R^2$ are as defined above, which is not labeled with $^{11}C$ (hereinafter to be referred to as compound (Ia)), or a salt thereof.

Compound (Ia) or a salt thereof can be produced by appropriately using the following Production Method 1a, a method of the above-mentioned Production Method 1 wherein $CH_3I$ is used instead of $^{11}CH_3I$, or organic chemical synthesis method known in the art.

(Production Method 1a)

(Scheme 1a)

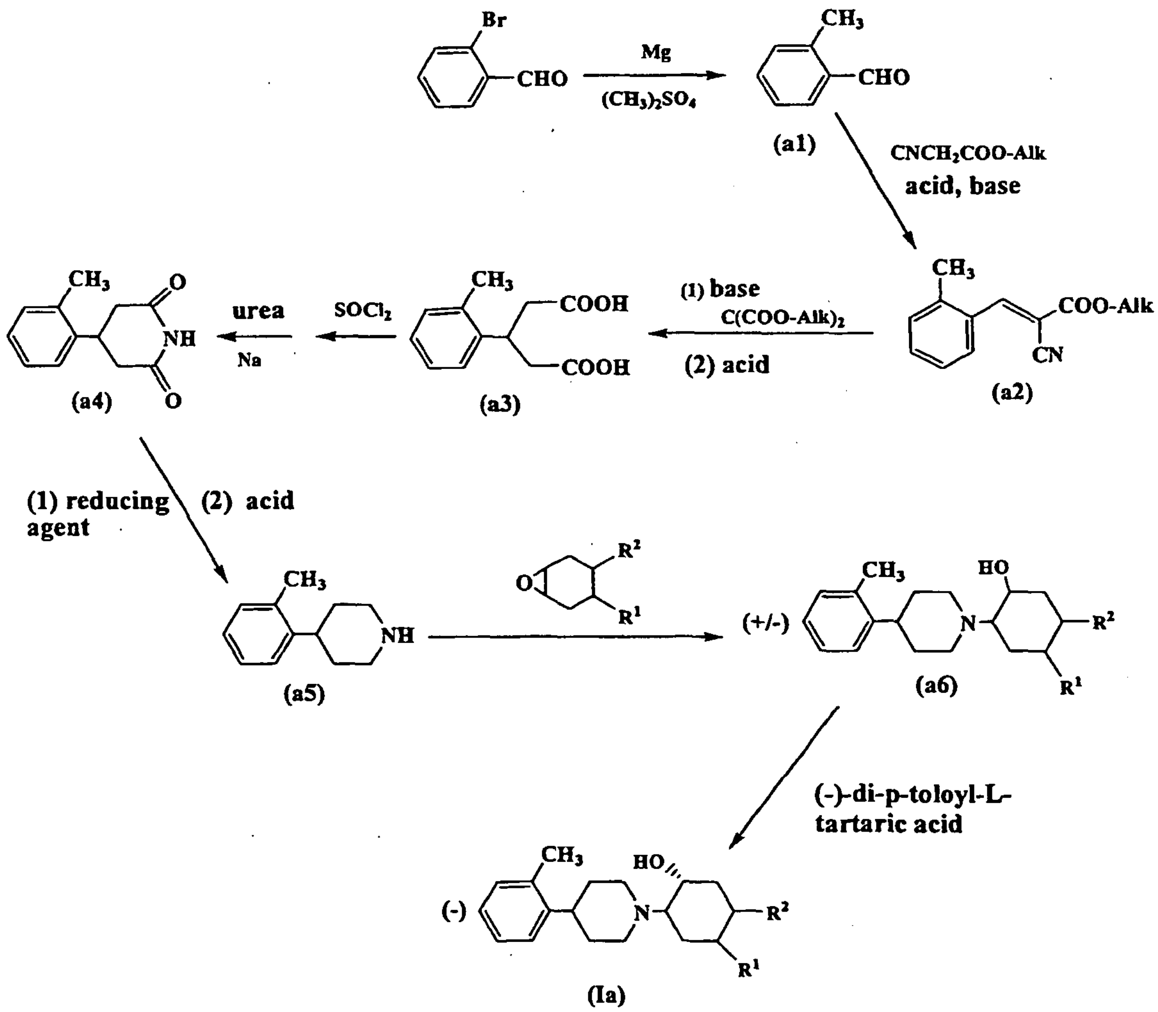


In each formula, $R^1$ and $R^2$ are as defined above, and the -Alk group means a lower alkyl group, preferably a $C_{1-6}$ alkyl group, more preferably an ethyl group. (synthesis of a compound represented by the formula (a1) (hereinafter to be referred to as compound (a1)))

Compound (a1) can be obtained by converting -Br group of 2-bromobenzaldehyde to $-CH_3$ group by a Grignard reaction. This reaction generally includes, under an inert gas (e.g., nitrogen, argon and the like) stream, adding dropwise 2-bromobenzaldehyde dissolved in an anhydride solvent to magnesium dissolved in an anhydride solvent, adding a catalytic amount of iodine, reacting the mixture for about 1 - 1.5 hr at any appropriate temperature between -15°C and 200°C according to the solvent to be used, adding dropwise dimethylsulfuric acid dissolved in an anhydride solvent, and reacting the mixture for about 1.5 - 2 hr at any appropriate temperature between -15°C and 200°C according to the solvent to be used.

The solvent to be used for this reaction is not particularly limited as long as it does not inhibit the reaction and, for example, THF, diethyl ether, dioxane, hexamethyl phosphine tri-amide (HMPA), benzene, a mixed solvent thereof and the like can be mentioned. As preferable solvent, THF can be mentioned.

The amount of the solvent to be used for this reaction is generally about 2- to 10-fold weight, preferably about 4- to 5-fold weight, relative to 2-bromobenzaldehyde.

The amount of magnesium to be used for this reaction is generally about 1.0- to 3.0-fold mole, preferably about 1.1-to 1.8-fold mole, relative to 2-bromobenzaldehyde.

The amount of dimethylsulfuric acid to be used for this reaction is generally about 1.0- to 5-fold mole, preferably about 1.2- to 3-fold mole, relative to 2-bromobenzaldehyde.

Where necessary, -CHO group of 2-bromobenzaldehyde may be protected and then the compound may be subjected to a Grignard reaction.

Preferably, 2-bromobenzaldehyde is reacted with ethylene glycol and p-toluenesulfonic acid in a solvent (e.g.,

benzene) for about 2 - 4 hr at any appropriate temperature between -15˚C and 200˚C according to the solvent to be used to give a ketal form, subjected to a Grignard reaction, and then deprotected using any appropriate acid (e.g., hydrochloric acid). (Synthesis of compound represented by the formula (a2) (hereinafter to be referred to as compound (a2)))

Compound (a2) is generally synthesized by reacting compound (a1) with $CNCH_2COO$-Alk wherein -Alk is as defined above in a suitable solvent in the presence of any appropriate acid (e.g., acetic acid) and a base (e.g., pyrrolidine).

The solvent to be used for this reaction is not particularly limited as long as it does not inhibit the reaction and, for example, benzene, toluene, THF, diethyl ether, acetonitrile, 1,4-dioxane and a mixed solvent thereof and the like can be mentioned. As preferable solvent, benzene can be mentioned.

The amount of the solvent to be used for this reaction is generally about 5- to 10-fold weight, preferably, about 7- to 9-fold weight, relative to compound (a1).

The amount of the acid to be used for this reaction is generally about 0.2- to 1.0-fold mole, preferably about 0.5- to 0.7-fold mole, relative to compound (a1).

The amount of the base to be used for this reaction is generally about 0.1- to 1.0-fold mole, preferably about 0.3-to 0.6-fold mole, relative to compound (a1).

The amount of $CNCH_2COO$-Alk wherein -Alk is as defined above to be used for this reaction is generally about 1.0- to 3.0-fold mole, preferably about 1.2- to 1.5-fold mole, relative to compound (a1).

The reaction temperature for this reaction is an appropriate temperature select from -15˚C to 200˚C according to the solvent to be used. The reaction time is generally about 1 - 5 hr, preferably about 2 - 3 hr.

(Synthesis of a compound represented by the formula (a3) (hereinafter to be referred to as compound (a3))

Compound (a3) is generally synthesized by reacting compound (a2) with $C(COO\text{-}Alk)_2$ wherein -Alk is as defined above in a suitable solvent in the presence of any appropriate base (e.g., NaH) under an inert gas (e.g., nitrogen, argon and the like) stream and then treating the compound with any appropriate acid(for example, hydrochloric acid).

The solvent to be used for this reaction is not particularly limited as long as it does not inhibit the reaction and, for example, THF, diethyl ether, 1,4-dioxane, benzene, acetonitrile, toluene and a mixed solvent thereof and the like can be mentioned. As preferable solvent, THF can be mentioned.

The amount of the solvent to be used for this reaction is generally about 3- to 10-fold weight, preferably, about 5- to 6-fold weight, relative to compound (a2).

The amount of the base to be used for this reaction is generally about 1.0- to 3.0-fold mole, preferably about 1.5-to 2.5-fold mole, relative to compound (a2).

The amount of $C(COO\text{-}Alk)_2$ wherein -Alk is as defined above to be used for this reaction is generally about 1.0- to 3.0-fold mole, preferably about 2.0- to 2.5-fold mole, relative to compound (a2).

The amount of the acid to be used for this reaction is generally about 20- to 50-fold mole, preferably about 30- to 40-fold mole, relative to compound (a2).

The reaction temperature for this reaction is an appropriate temperature select from -15˚C to 200˚C according to the solvent to be used. The total reaction time is about 50 - 90 hr, preferably about 70 - 80 hr.

(Synthesis of compound represented by the formula (a4) (hereinafter to be referred to as compound (a4)))

Compound (a4) is generally synthesized by reacting compound (a3) with thionyl chloride in an anhydride solvent, once evaporating the solvent, adding urea, adding sodium in the anhydride solvent and refluxing the mixture.

The solvent to be used for this reaction is not particularly limited as long as it does not inhibit the reaction and, for example, methanol, ethanol, isopropyl alcohol, acetonitrile and a mixed solvent thereof and the like can be mentioned. As preferable solvent, ethanol can be mentioned.

The amount of the solvent to be used for this reaction is generally about 2- to 10-fold weight, preferably, about 4- to 5-fold weight, relative to compound (a3).

The amount of thionyl chloride to be used for this reaction is generally about 2- to 10-fold mole, preferably about 3- to 5-fold mole, relative to compound (a3).

The amount of urea to be used for this reaction is generally about 1.1- to 5.0-fold mole, preferably about 2.0- to 3.0-fold mole, relative to compound (a3).

The amount of sodium to be used for this reaction is generally about 1.1- to 5.0-fold mole, preferably about 2.0-to 3.0-fold mole, relative to compound (a3).

The reaction temperature for this reaction is an appropriate temperature select from -15˚C to 200˚C according to the solvent to be used. The reaction time is generally about 10 - 24 hr, preferably, about 20 - 24 hr.

(Synthesis of compound represented by the formula (a5) (hereinafter to be referred to as compound (a5)))

Compound (a5) is generally synthesized by, under ice-cooling, adding compound (a4) to the reducing agent (e.g., $BH_3$) in the solvent, refluxing the mixture, after which adding, where necessary, any appropriate acid (e.g., hydrochloric acid), and further refluxing the mixture.

The solvent to be used for this reaction is not particularly limited as long as it does not inhibit the reaction and, for example, THF, diethyl ether, 1,4-dioxane and a mixed solvent thereof and the like can be mentioned. As preferable solvent, THF can be mentioned.

The amount of the solvent to be used for this reaction is generally about 10- to 20-fold weight, preferably, about 12-to 15-fold weight, relative to compound (a4).

The amount of the reducing agent to be used for this reaction is generally about 1.1- to 10-fold weight, preferably, about 2- to 5-fold weight, relative to compound (a4).

(Synthesis of compound represented by the formula the formula (a6) (hereinafter to be referred to as compound (a6)) and compound (Ia))

Synthesis of compound (a6) from compound (a5), and further from compound (a6) to compound (Ia) can be performed according to Steps a - e shown in Scheme 1-1 of the aforementioned Production Method 1.

As the salt of compound (Ia), those exemplified for the aforementioned compound (I) can be mentioned. Compound (Ia) can be converted to a pharmaceutically acceptable salt thereof by method known in the art.

Compound (Ia) and a salt thereof have a specific binding affinity for VAChT, and therefore, are useful as a tool for studying VAChT, for example, a reagent (e.g., tracer and the like) for studying the function of VAChT and the like.

**Examples**

The present invention is explained in more detail in the following by referring to Examples, which are mere examples of the present invention and should not be construed as limitative. The unit "M" means mol/L.

<u>(Example 1: Synthesis of (-)-2-(4-(2-methylphenyl)piperidino)cyclohexanol (hereinafter sometimes to be abbreviated as "(-)-o-methylvesamicol" or "(-)-o-MeVES"))</u>

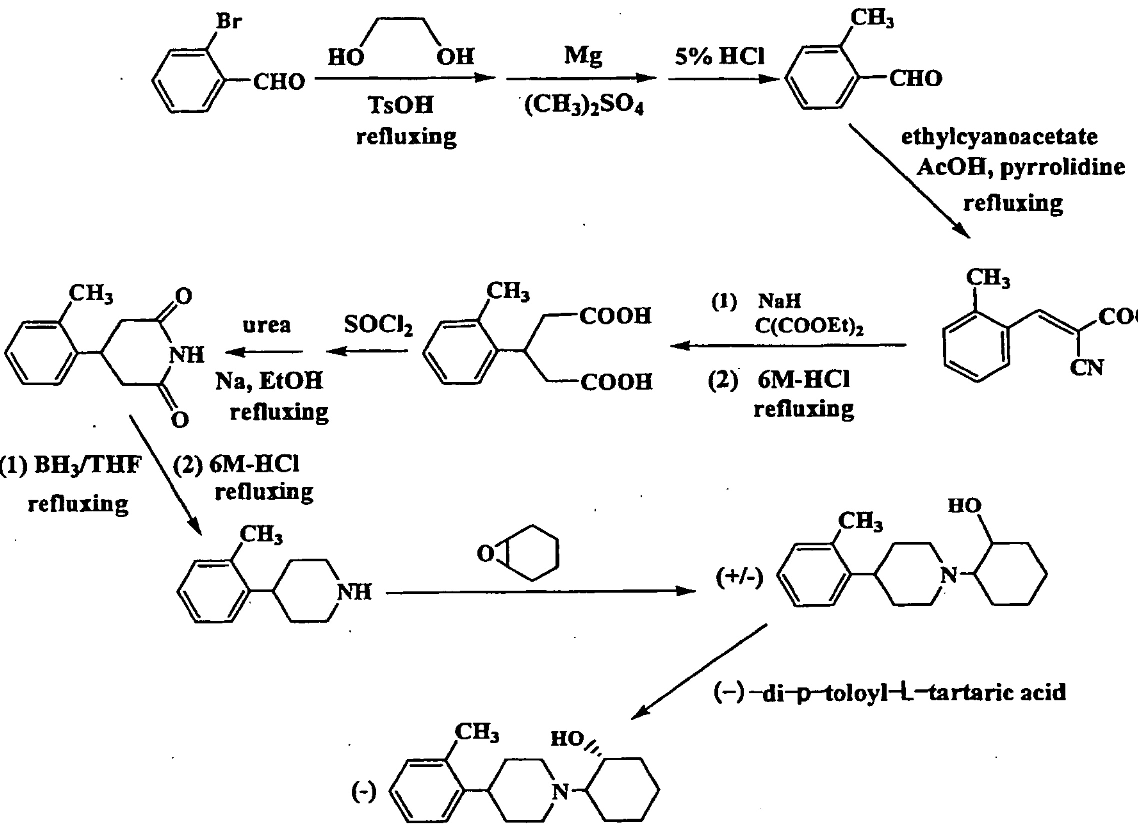


(step A: Synthesis of 2-methylbenzaldehyde)

2-Bromobenzaldehyde (25 g, 135 mmol), ethylene glycol (41.9 g, 675 mmol) and p-toluenesulfonic acid (2.57 g, 13.5 mmol) were added to benzene (300 mL), and the mixture was refluxed for 3 hr to give a ketal form. Under an argon stream, magnesium (3.64 g, 150 mmol) was added to dry THF (50 mL) and the mixture was stirred. Then, a solution (50 mL) of the ketal form in dry THF was added dropwise, a catalytic amount of iodine was added, and the mixture was refluxed for 1 hr. A solution of dimethylsulfuric acid (37.8 g, 300 mmol) in dry THF (60 mL) was added dropwise, and the mixture was further refluxed for 1.5 hr to perform methylation ($-CH_3$) of bromine (-Br) moiety of 2-bromobenzaldehyde. The produced methyl form was deketalized with 5% hydrochloric acid to give the title compound as an oil (yield from 2-bromobenzaldehyde, 70.8%).
$^1$H-NMR ($CDCl_3$) :δ 10.26 (bs, 1H), 7.84-7.31 (m, 4H), 2.66 (s, 3H)
Mass (m/e) : 129($M^+$)

(step B: Synthesis of ethyl 2-cyano-3-(2-methylphenyl)acrylate)

2-Methylbenzaldehyde (17.8 g, 148 mmol) and ethylcyanoacetate (20.1 g, 177.7 mmol) were added to benzene (150 mL), acetic acid (5.6 mL) and pyrrolidine (3.07 mL) were added, and the mixture was refluxed for about 2 hr to give the title compound as a white solid (yield 55.3%).
$^1$H-NMR($CDCl_3$): δ 8.56 (s, 1H), 8.18-8.09 (m, 1H), 7.38-7.34(m, 3H), 4.58-4.22(dd, J= 7.02 Hz, J= 14.3 Hz, 2H), 2.45 (s, 3H), 1.52-1.29 (t, J= 7.02Hz, 3H)
Mass (m/e) : 215 ($M^+$)

(step C: Synthesis of 4-(2-methylphenyl)piperidine-2,6-dione)

Under an argon stream, to dry THF (25 mL) containing sodium hydride (3.18 g, 79.6 mmol) was added diethylmalonate (12.96 g, 81 mmol), and the mixture was stirred at room temperature for 15 min. Then, a solution of ethyl 2-cyano-3-(2-methylphenyl)acrylate (8.56 g, 39.8 mmol) in dry THF (50 mL) was added dropwise thereto, and the mixture was stirred at room temperature for 15 hr. After completion of the reaction, the reaction mixture was further refluxed in 6M-hydrochloric acid (200 mL) for 60 hr. After completion of the reaction, the obtained 3-(2-methylphenyl)pentane-1,5-diacid (13 g, 58.6 mmol) and thionyl chloride (21.62 g, 13.3 mmol) were added to ice-cooled anhydrous methanol (90 mL), and the mixture was reacted by stirring for 60 min. After completion of the reaction, the solvent was evaporated, and the residue was washed with saturated sodium hydrogencarbonate solution and dried over anhydrous magnesium sulfate. The oil obtained by evaporating the solvent and urea (5.28 g, 87.9 mmol) were added to anhydrous ethanol (50 mL), sodium (2.03 g, 87.9 mmol) was added and the mixture was refluxed for 19 hr. After completion of the reaction, the reaction mixture was cooled and filtered to give the title compound as a solid (yield from ethyl 2-cyano-3-(2-methylphenyl)acrylate, 64.1%).
$^1$H-NMR($CD_3OD$): δ 7.19 (s, 4H), 2.83-2.70 (d, 4H), 3.92-3.61 (m, 1H), 2.36 (s, 3H)
Mass(m/e): 203 ($M^+$)

(step D: Synthesis of 4-(2-methylphenyl)piperidine)

Under ice-cooling, 4-(2-methylphenyl)piperidine-2,6-dione (9.46 g, 46.6 mmol) was added to 1M-$BH_3$-THF (150 mL), and the mixture was refluxed for 19 hr. Then, 6M-hydrochloric acid (100 ml) was added, and the mixture was further refluxed for 22 hr to give the title compound as a solid (yield 81.0%).
$^1$H-NMR($CD_3OD$): δ 7.19 (s, 4H), 4.15-3.54 (m, 5H), 2.35 (s, 3H), 2.05 (m, 4H)
Mass(m/e): 175 ($M^+$)

(step E: Synthesis of (-)-2-(4-(2-methylphenyl)piperidino)cyclohexanol)

4-(2-Methylphenyl)piperidine (4.65 g, 26.6 mmol) and cyclohexeneoxide (9.8 g, 101 mmol) were added to anhydrous ethanol (30 mL), and the mixture was refluxed for 20 hr to give (±)-2-(4-(2-methylphenyl)piperidino)cyclohexanol((±)-o-methylvesamicol) (yield 31.9%). Then, the obtained (±)-o-methylvesamicol (1.63 g, 6.0 mmol) was dissolved in acetone (25 mL), a solution of (-)-di-p-toluoyl-L-tartaric acid (2.54 g, 6.56 mmol) in acetone (25 mL) was added dropwise, and the mixture was stirred at room temperature for 5 min. The precipitated crystals were passed through a glass filter. The crystals in the filter were dissolved in 2M-sodium hydroxide solution, and the solution was stirred for 5 min. After stirring, the mixture was extracted with a mixed solvent of methylene chloride and 5% methanol, and dried over anhydrous magnesium sulfate. The solvent was evaporated and the residue was recrystallized from ethanol to give the title compound as a solid (yield 66.5%).
Mp: 116-118°C
$^1$H-NMR($CD_3OD$): δ 7.25-7.09 (m, 4H), 3.70-3.42 (m, 1H), 2.86-2.69 (m, 3H), 2.33 (s, 3H), 2.33-2.07 (m, 3H), 1.84-1.71 (m, 8H), 1.38-1.19 (m, 4H)
Mass(m/e): 273 (M+)
Anal. Calcd. For $C_{18}H_{27}NO$: C, 79.07; H, 9.95; N, 5.12. Found: C, 78.88; H, 10.15; N, 5.06

(Example 2: Comparison of affinity of (-)-o-MeVES for VAChT and affinity for sigma receptors (σ-1 and σ-2) in vitro)

The affinity of (-)-o-MeVES for VAChT and sigma receptors (σ-1, σ-2) was examined by in vitro drug inhibition experiments.

(Preparation of rat cerebral membranes and liver membranes)

First, the brain and liver were extirpated from a rat, a 0.32 M sucrose solution in an amount by weight 10 times the weight of each tissue was added, and each tissue was ground using a Teflon homogenizer. Next, the ground tissue solution was centrifuged at 1000 g for 10 minutes, and the precipitated fraction was removed. Furthermore, the supernatant was centrifuged at 55000g for 60 minutes, and the precipitated tissue was added to, and suspended in, 50 mM Tris-HCl buffer solution/0.32 M sucrose solution (pH 7.8).

(Test of affinity for VAChT)

10 nM (-)-[$^3$H]vesamicol was used as the radioisotope (RI) for VAChT. As the test compounds, (-)-o-MeVES, (-)-vesamicol, 1-(3,4-dimethoxyphenethyl)-4-(3-phenyl-propyl)piperazine (hereinafter referred to as SA4503), haloperidol, and (+)-pentazocine, shown in Table 1-1, were used. For each test compound, samples at 10 different concentrations (concentrations set at 10 levels in the range of $10^{-5}$ M to $10^{-10}$ M) were prepared.
For each sample, the RI (100 μl), sample (50 μl), 50 mM Tris-HCl buffer solution (pH 7.8) (100 μl), 2 μM DTG solution (50 μl) (200 nM as the final concentration), and, finally, the prepared rat cerebral membrane (500 - 900 μg (protein weight)/200 μl) was added to a test tube, and they were reacted at 37°C for 60 minutes. The test tube was cooled with ice to stop the reaction, and the rat cerebral membrane was immediately adsorbed to a glass filter using a

cell harvester. After the glass filter was washed with 50 mM Tris-HCl buffer solution (pH 7.8) (5 ml) three times, the rat cerebral membrane, together with the glass filter, was placed in a liquid scintillation vial, and a liquid scintillator was added, after which radioactivity was measured using a liquid scintillation counter.

To determine the amount bound non-specifically, a sample to which 10 μM vesamicol had been added was tested concurrently.

(Test of affinity for σ-1 receptor)

A test was performed in the same procedures as those for the test of affinity for VAChT except that the reaction was performed at 37˚C for 90 minutes using 3 nM (+)-[$^3$H]pentazocine as the radioisotope (RI) for the σ-1 receptor.

To determine the amount bound non-specifically, a sample to which 10 μM pentazocine had been added was tested concurrently.

(Test of affinity for σ-2 receptor)

3 nM [$^3$H]DTG ([$^3$H]-1,3-di-o-tolylguanidine) was used as the radioisotope (RI) for the σ-2 receptor. To suppress the binding of this RI to the σ-1 receptor, 1 μM pentazocine (50 μl) was added, and 50 mM Tris-HCl buffer solution (pH 7.8) (100 μl) was added. A test was performed in the same procedures as those for the test of affinity for the σ-1 receptor except that rat liver membranes (300 - 600 μg (protein weight)/200 μl) were used.

To determine the amount bound non-specifically, a sample to which 10 μM DTG and 1 μM pentazocine had been added was tested concurrently.

The results are shown in Table 1-1.

[Table 1-1]

| | $IC_{50}$ (nM) | | |
|---|---|---|---|
| | VAChT* | σ-1 Receptor# | σ-2 Receptor$ |
| (-)-o-MeVES | 16 | 38 | 320 |
| (-)-Vesamicol | 13 | 130 | 420 |
| SA4503 | 120 | 5.1 | 280 |
| Haloperidol | 97 | 3.0 | 190 |
| (+)-Pentazocine | 830 | 6.3 | - |
| N = 3 (mean for three experiments) | | | |

Each value in the Table is shown as the mean value for three experiments.

*: Rat cerebral membranes, in the presence of 200 nM DTG for masking the two sigma receptors, were incubated at 37˚C for 60 minutes, with (-)-[$^3$H]vesamicol in 50 mM Tris-HCl buffer solution (pH 7.8).

#: Rat cerebral membranes were incubated at 37˚C for 90 minutes, with (+)-[$^3$H]pentazocine in 50 mM Tris-HCl buffer solution (pH 7.8).

$: Rat liver membranes, in the presence of 0.001 mM (+)-pentazocine for masking the σ-1 receptor, were incubated at 37˚C for 90 minutes, with [$^3$H]DTG in 50 mM Tris-HC1 buffer solution (pH 7.8).

Next, on the basis of the above-described experimental results (n=3) and the results of five experiments performed in the same manner as the above-described experiment (n=5), Ki values were obtained (n=8 in total). Ki values are generally used to correct affinity ($IC_{50}$ value) data variation due to condition differences among individual experiments, whereby a comparison of, for example, affinity data obtained from different experimental days and affinity data on different receptors (for example, VAChT and δ-1 receptor) can be performed.

The Ki values were calculated according to the calculation formula shown below. The results are shown in Table 1-2.

$$\text{formula: } Ki = IC_{50}/(1+C/Kd)\ (nM)$$

(wherein Kd represents the dissociation constant of the radioactive ligand, and C represents the concentration of the radioactive ligand.)

[Table 1-2]

| | Ki(nM) | | |
|---|---|---|---|
| | VAChT | δ-1 receptor | δ-2 receptor |
| (-)-o-MeVES | 6.7 ± 0.4 | 33.7 ± 5.9 | 266 ± 28 |
| (-)-vesamicol | 4.4 ± 0.6 | 73.8 ± 11.2 | 346 ± 37 |
| SA4503 | 50.2 ± 7.2 | 4.4 ± 1.0 | 242 ± 17 |
| haloperidol | 41.4 ± 17.6 | 2.6 ± 0.8 | 167 ± 19 |
| (+)-pentazocine | 315 ± 121 | 5.5 ± 2.0 | - |
| N = 8 (mean for a total of 8 experiments, including supplementary experiments) | | | |

Each value in the Table is shown as mean±standard deviation.

As shown in Table 1-2, the Ki value indicating the affinity of (-)-vesamicol for VAChT is 4.4±0.6 nM, and the Ki value indicating the affinity of the (-)-o-MeVES of the present invention for VAChT is 6.7±0.4 nM. Also, the Ki values of the affinity of (-)-vesamicol for the δ-1 receptor and δ-2 receptor are 73.8±11.2 nM and 346±37 nM, respectively, and the Ki values indicating the affinity of the (-)-o-MeVES of the present invention for the δ-1 receptor and δ-2 receptor are 33.7±5.9 nM and 266±28 nM, respectively. From this result, (-)-vesamicol and the (-)-o-MeVES of the present invention were found to have higher affinity for VAChT and lower affinity for the δ-1 receptor and δ-2 receptor compared to other ligands (SA4503, haloperidol and (+)-pentazocine).

From these results, it can be said that the (-)-o-MeVES of the present invention maintains the properties of (-)-vesamicol, i.e., high affinity for VAChT and low affinity for the δ-1 receptor and δ-2 receptor. Furthermore, the (-)-[$^{11}$C]-o-MeVES of the present invention can be said to be useful as a diagnostic reagent for a diagnostic method with VAChT as an index because it has the $^{11}$C-label while retaining the above-described properties of (-)-vesamicol.

In the aforementioned Life Sciences 71 (2002) 1591-1598, it is described that as a result of in vitro experiments using the rat brain, the affinity of (-)-o-iodovesamicol for VAChT, δ-1 receptor, and δ-2 receptor were 15.0±2.9 nM, 62.2±12.0 nM, and 554±137 nM, respectively, as Ki values (mean±standard deviation). On the other hand, from Table 1-2, the Ki values of the (-)-o-MeVES of the present invention for VAChT, δ-1 receptor, and δ-2 receptor were 6.7+0.4 nM, 33.7±5.9 nM, and 266±28 nM, respectively. Hence, both (-)-o-MeVES and (-)-o-iodovesamicol have high affinity for VAChT and low affinity for the δ-1 receptor and δ-2 receptor; comparing them, the affinity of (-)-o-MeVES for VAChT is still higher than (-)-o-iodovesamicol. Also, the δ-1 receptor/VAChT ratio and δ-2 receptor/VAChT ratio of the Ki values are higher for (-)-o-MeVES than for (-)-o-iodovesamicol; the former is better in terms of specificity for VAChT.

Also, (-)-o-iodovesamicol can be expected for use in SPECT, as stated above, but the (-)-o-MeVES and (-)-[$^{11}$C]-o-MeVES of the present invention are useful in that they can be used in PET, which is better than SPECT in terms of sensitivity, resolution, and quantitative analyzability.

From these results, it was shown that in (-)-o-MeVES, methyl group introduction to the ortho-position of the benzene ring moiety of (-)-vesamicol did not influence the high affinity of (-)-vesamicol for VAChT and the low affinity for the two sigma receptors. As a result, (-)-o-MeVES was shown to be a VAChT ligand as good as (-)-vesamicol.

<u>(Example 3: Synthesis of (-)-2-(4-(2-trimethyl stanyl phenyl)piperidino)cyclohexanol (hereinafter to be abbreviated as "(-)-o-trimethyl stanyl vesamicol"))</u>

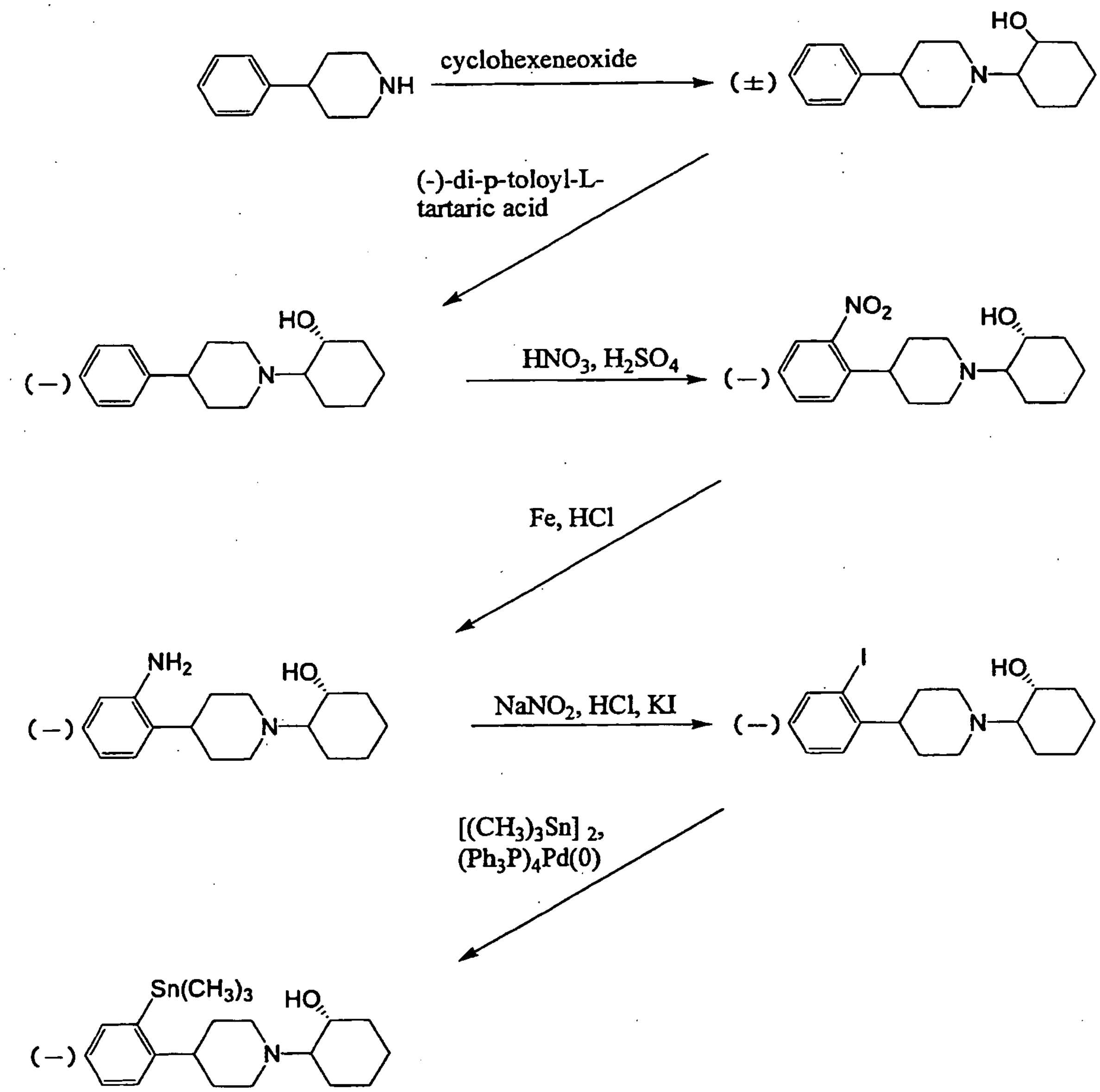


(-)-o-Iodovesamicol was obtained according to the method described in Shiba et al., Life Sciences 71 (2002) 1591-1598 and Shiba et al., Annals of Nuclear Medicine Vol. 17, No.6, 451-456, 2003.

(-)-o-Iodovesamicol (116 mg, 0.3 mmol), hexamethylditin (246 mg, 0.75 mmol) and tetrakis(triphenylphosphine) palladium(0) (20 mg, 17.5 μmol) were added to anhydrous toluene (5 mL), and the mixture was stirred under an argon stream for 5 min and further refluxed for 15 hr. The solvent was evaporated from the reaction mixture, and the residue was purified by preparative thin layer chromatography (solvent:ethyl acetate/hexane=1:5) to give the title compound as a colorless oil (yield 33.0%).

$^1$H-MNR($CDCl_3$) : δ 0.30 (9H, s), 1.42-1.60 (4H, m), 1.60-1.95 (8H, m), 2.02-2.40 (3H, m), 2.62-3.00 (3H, m), 4.22-4.68 (1H, m), 6.95-7.50 (4H, m)

Mass (m/e): 421,423 $[M]^+$

<u>(Example 4: Synthesis of (-)-[$^{11}$C]-2-(4-(2-methylphenyl)piperidino)cyclohexanol (hereinafter to be abbreviated as "(-)-[$^{11}$C]-o-methylvesamicol"))</u>

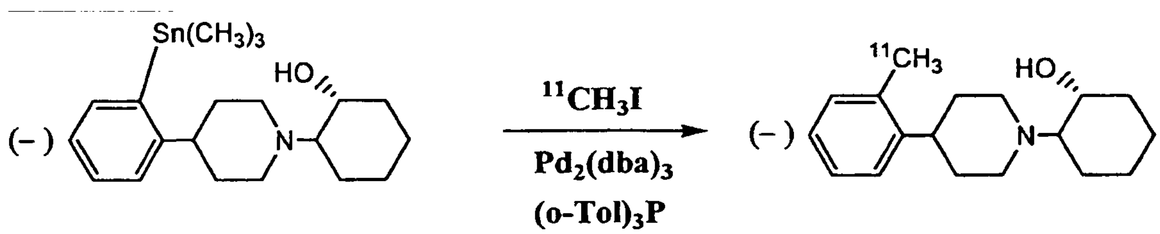


Tris (dibenzylideneacetone) dipalladium ($Pd_2(dba)_3$) (2.8 mg, 3.0 μmol) and tri-o-toluylphosphine ($(o\text{-}Tol)_3P$) (3.7 mg, 120 μmol) were weighed, dissolved in DMF, and placed in a reaction vessel (RV1) of the apparatus shown in Fig. 1. CuCl (1.2 mg, 12 μmol), $K_2CO_3$ (0.6 mg, 4.3 μmol), and (-)-o-trimethylstanylvesamicol (0.6 mg, 1.5 μmol) obtained in Example 3 were dissolved in DMF (0.3 ml) and placed in a first syringe (VA1) of the apparatus shown in Fig. 1. $^{11}CH_3I$ ([$^{11}$C]methyl iodide (10 - 50 GBq as radioactivity amount)) was introduced into a reaction vessel (RV1) of the apparatus shown in Fig. 1 at room temperature and [$^{11}$C]methyl iodide was reacted with the above-mentioned reagent for 1 min after the introduction. Then, the solution containing (-)-o-trimethylstanylvesamicol in the first syringe (VA1) was introduced into the reaction vessel (RV1) and a methylation reaction was performed at 80°C for 3 min. The reaction vessel was cooled, the HPLC eluent in the second syringe (VA2) was added to the reaction solution using $N_2$ gas, and the reaction solution was filtered with a glass filter having a diameter of 13 mm and a pore size of 0.7 μm. The obtained filtrate was separated by preparative HPLC under the following conditions.

The solvent was evaporated from the HPLC fraction containing (-)-[$^{11}$C]-o-methylvesamicol, and saline was added to give a preparation for injection. The radiochemical yield of the title compound was 38% relative to [$^{11}$C]methyl iodide. The results of labeling of (-)-[$^{11}$C]-o-methylvesamicol as analyzed by analysis HPLC under the following conditions are shown in Table 2.

conditions for preparative HPLC
YMC Pack ODS-A 10x250 mm (JASCO Corporation)
$CH_3CN$:50 mM $AcONH_4$ (2.51 g/650 mL $H_2O$)= 350:650 flow rate = 5 mL/min, UV wavelength = 260 nm
conditions for HPLC analysis
Finepak SIL C18S 4.6x250 mm (JASCO Corporation)
$CH_3CN$:30 mM $AcONH_4$:AcOH = 500:500:2
flow rate = 2 mL/min, UV wavelength = 254 nm

[Table 2]

| | | (-)-[$^{11}$C]-o-MeVES |
|---|---|---|
| production amount (yield) | MBq (%) | 600(38) |
| radio chemical purity | % | >99 |
| relative radioactivity | GBq/μmol | 52.4 |
| reaction time | min | 3 |
| reaction temperature | °C | 80 |
| synthesis time | min | 30 |

(Example 5: Tissue distribution of radioactivity after intravenous injection of (-)-[$^{11}$C]-o-methylvesamicol in rats)

From the tail vein of each rat, an injection containing 20 MBq of (-)-[$^{11}$C]-o-MeVES (0.2 ml) was administered. At 5 minutes, 15 minutes, 30 minutes, and 60 minutes after administration, the rats were killed by cervical dislocation, and blood was drawn from the heart using a syringe, after which tissues such as the heart, lungs, liver, and brain were extirpated, and the radioactivity in each tissue was measured using a gamma counter. Each tissue was also weighed.

The results are shown in Table 3.

[Table 3]

| | Radioactivity level | | | |
|---|---|---|---|---|
| Tissue | 5 min | 15 min | 30 min | 60 min |

(continued)

| | Radioactivity level | | | |
|---|---|---|---|---|
| blood | 0.45±0.04 | 0.27±0.03 | 0.24±0.02 | 0.18±0.02 |
| heart | 3.37±0.30 | 1.82±0.19 | 1.52±0.11 | 0.96±0.02 |
| lung | 16.55±2.56 | 10.38±2.48 | 8.74±0.34 | 4.81±0.76 |
| liver | 2.58±0.23 | 2.93±0.38 | 3.22±0.76 | 3.28±0.32 |
| pancreas | 8.44±1.93 | 12.13±2.23 | 11.53±2.67 | 10.67±1.22 |
| spleen | 4.97±1.06 | 5.90±0.51 | 5.56±0.52 | 4.24±0.35 |
| kidney | 11.10±4.31 | 7.52±0.40 | 6.02±0.09 | 4.15±0.50 |
| small intestine | 6.19±0.58 | 8.76±1.66 | 8.48±1.85 | 8.90±0.50 |
| muscle | 1.57±0.27 | 1.42±0.44 | 1.18±0.24 | 1.00±0.16 |
| brain | 5.47±0.44 | 4.54±0.54 | 4.71±0.45 | 3.86±0.28 |
| *Radioactivity levels are shown as mean % ± S.D. (n=4 - 6) of radioactivity per gram of tissue to the total radioactivity administered. | | | | |

The results in Table 3 show the distribution of (-)-[$^{11}$C]-o-MeVES in the rat body. By this data, the extent of radioactivity accumulation in the brain, that is, the possibility of brain imaging, can be known. It is seen that the brain accumulation is retainable, that the radioactivity is rapidly cleared from the blood, and that (-)-[$^{11}$C]-o-MeVES is suitable for imaging. Also, because (-)-[$^{11}$C]-o-MeVES is a radioactive compound, it is necessary to take into consideration exposure in the body during clinical use; however, from the results in Table 3, regarding the (-)-[$^{11}$C]-o-MeVES of the present invention, there are no organs showing particularly high accumulation or organs showing extremely increased accumulation over time, and there are no organs showing particularly high exposure doses, and therefore it can be thought that there is no problem with exposure.

(Example 6: Inhibitory effects of (-)-o-methylvesamicol, (-)-vesamicol, and sigma receptor ligands on cerebral distribution of (-)-[$^{11}$C]-o-methylvesamicol in rats)

Each of (-)-o-methylvesamicol, (-)-vesamicol, SA4503, haloperidol, and (+)-pentazocine, as non-radioactive ligands, was dissolved in DMSO to obtain a concentration of 1250 nmol/ml.

To the caudal vein of each rat, an injection (0.1 ml) containing 20 MBq of (-)-[$^{11}$C]-o-MeVES and 0.1 ml (equivalent to 500 nmol per kg body weight) of a DMSO solution containing each of the above-described non-radioactive ligands were administered together. Thirty minutes after the administration, the rats were killed by cervical dislocation, and blood was drawn from the heart using a syringe, after which the brain was extirpated from each rat. The extirpated brain was divided into the cerebral cortex, hippocampus, striate body, cerebellum, medulla oblongata, and other parts, and the radioactivity in each tissue was measured using a gamma counter. Each tissue was also weighed.

For control, an injection (0.1 ml) containing 20 MBq of (-)-[$^{11}$C]-o-MeVES and a ligand-free DMSO solution (0.1 ml) were administered to the caudal vein of each rat, and measurements were taken in the same manner as described above.

The results are shown in Table 4.

[Table 4]

| | Radioactivity level (% ID/g) | | | | | |
|---|---|---|---|---|---|---|
| | Control | (-)-o-Methylvesamicol | (-)-Vesamicol | SA4503 | Haloperidol | (+)-Pentazocine |
| Cerebral cortex | 2.58±0.26 | 0.70±0.10 | 0.71±0.04 | 0.62±0.08 | 1.03±0.05 | 2.72±0.40 |
| Hippocampus | 2.40±0.30 | 0.71±0.08 | 0.66±0.05 | 0.84±0.18 | 1.61±0.15 | 2.59±0.44 |
| Striate body | 2.14±0.18 | 0.71±0.10 | 0.72±0.03 | 0.61±0.07 | 1.04±0.10 | 2.39±0.40 |
| Cerebellum | 2.18±0.23 | 0.60±0.07 | 0.62±0.03 | 0.52±0.07 | 0.92±0.04 | 2.07±0.31 |

(continued)

| | Radioactivity level (% ID/g) | | | | | |
|---|---|---|---|---|---|---|
| | Control | (-)-o-Methylvesamicol | (-)-Vesamicol | SA4503 | Haloperidol | (+)-Pentazocine |
| Medulla oblongata | 2.68±0.23 | 0.74±0.09 | 0.80±0.08 | 0.66±0.08 | 1.10±0.05 | 2.83±0.45 |
| Brain (other than above) | 2.40±0.23 | 0.63±0.10 | 0.69±0.04 | 0.55±0.05 | 0.95±0.06 | 2.52±0.43 |
| Brain (whole) | 2.48±0.23 | 0.68±0.10 | 0.70±0.04 | 0.59±0.07 | 1.03±0.04 | 2.52±0.40 |
| Blood | 0.11±0.01 | 0.14±0.02 | 0.13±0.01 | 0.14±0.02 | 0.16±0.01 | 0.12±0.01 |
| *Radioactivity levels are shown as mean % ± S.D. (n=4 - 6) of radioactivity per gram of tissue to the total radioactivity administered. | | | | | | |

From these results, the local accumulation of (-)-[$^{11}$C]-o-MeVES in the brain was inhibited by non-radioactive (-)-o-methylvesamicol and (-)-vesamicol, which have high affinity for VAChT, and by SA4503 and haloperidol, which have low affinity for VAChT. On the other hand, the accumulation was not inhibited by (+)-pentazocine, which is selective for the sigma receptors. From this, it was shown that (-)-[$^{11}$C]-o-MeVES binds specifically to VAChT in the brain.

(Example 7: Rat brain autoradiography using (-)-[$^{11}$C]-o-methylvesamicol)

To the caudal vein of each rat, 110 - 120 MBq of (-)-[11C]-o-MeVES was administered, and the rats were killed by cervical dislocation and had the brain extirpated 15 minutes after administration for the first rat, or 30 minutes after administration for the second rat. The extirpated brain was immediately frozen with dry ice, and a brain section 20 μm in thickness was prepared using a cryomicrotome. The brain section was mounted on a glass plate, this section was dried on a hot plate (60˚C), and images of radioactivity distribution were obtained by radioluminography. The results are shown in Figure 2.

From these results, the local distribution of (-)-[$^{11}$C]-o-MeVES in the brain agreed well with the in vitro local distribution of (-)-[$^{3}$H]-vesamicol masking the sigma receptors in the brain, while showing a distinct difference from the distribution of [$^{11}$C]SA4503, which reflects the sigma receptors; it was shown that (-)-[$^{11}$C]-o-MeVES is an in vivo ligand capable of imaging VAChT in the brain.

Here, VAChT is abundantly present in the hippocampus, cerebellum, and motor nerve nucleus, and is also relatively abundantly present in the striate body, cortex, and thalamus/hypothalamus.

The images shown in Figure 2 can be said to indicate that the cerebral distribution of the (-)-[$^{11}$C]-o-MeVES of the present invention reflects the distribution of VAChT in the rat brain.

In the case of diseases considered to be involved by the cholinergic nervous system, generally, changes in the activity, quantity and the like of VAChT are seen. Brain PET images obtained with the (-)-[$^{11}$C]-o-MeVES of the present invention are capable of sensitively visualizing changes in the activity, quantity and the like of VAChT as density differences (or color tone differences) in the images, and are also capable of quantifying the changes by image analysis. Therefore, it is possible to visually diagnosing diseases such as cholinergic neurodegenerative disorders (for example, Alzheimer's disease, memory disturbance, learning disability, schizophrenia, cognitive functional disorder, hyperkinetic disorder, anxiety neurosis, depression, analgesia, Parkinson's disease and the like) using the (-)-[$^{11}$C]-o-MeVES of the present invention. Also, because changes in VAChT can be quantified, degree of progression of disease can be known from the degree of the change, and early diagnosis is possible.

(Example 8: Comparison of PET brain images of learning disability model monkeys and healthy monkeys using (-)-[$^{11}$C]-o-methylvesamicol)

185 - 740 MBq of (-)-[$^{11}$C]-o-MeVES was administered to a vein of each of learning disability model monkeys (neurolysis monkeys) created by selectively destroying the p75NTR positive cells (ACh neurons) in the right hemisphere of the brain with 192-Saporin (manufactured by Advanced Targeting Systems) and healthy monkeys, and image data were collected using PET for animals (PET; manufactured by Hamamatsu Photonics K.K., SHR-7700) at 10 seconds to 91 minutes after administration. The image data obtained at 15 minutes to 60 minutes after administration were picturized; the photographs thus obtained are shown in Figure 4. In the images obtained at 15 minutes to 60 minutes after administration, a significant reduction in RI accumulation was observed in the cerebral cortex on the neurolysis

side (right hemisphere of the brain) of the neurolysis monkeys, compared to the healthy side. On the other hand, in the healthy monkeys, no change in the accumulation was observed.

From these results, it was shown that (-)-[$^{11}$C)-o-MeVES is capable of sensitively picturizing VAChT changes by PET-based in vivo imaging using monkeys, and can serve as a diagnostic reagent for diagnosing cholinergic neurodegenerative disorders and the like.

## Industrial Applicability

Compound (I) of the present invention or a salt thereof can be used as a reagent for VAChT mapping using PET and the like. Compound (I) or a salt thereof is also useful as a diagnostic reagent and the like for diagnosing cholinergic neurodegenerative disorders (e.g., Alzheimer's disease, memory disorder, learning disorder, schizophrenia, cognitive dysfunction, hyperactivity disorder, anxiety neurosis, depression, analgia, Parkinson's disease and the like) characterized by a decrease in VAChT. Moreover, Compound (II) of the present invention or a salt thereof is useful as a precursor for producing the above-mentioned reagent and diagnostic reagent, and the like. Furthermore, compound (Ia) of the present invention or a salt thereof is useful as a tool for studying VAChT and the like.

This application is based on a patent application No. 2005-106824 filed in Japan (filing date: April 1, 2005), the contents of which are incorporated in full herein by this reference.

## Claims

1. A compound represented by the following formula (I):

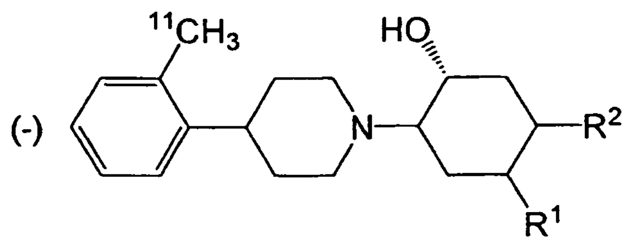

(I)

wherein $R^1$ and $R^2$ are the same or different and each is a hydrogen atom, a halogen atom, a hydroxyl group, an amino group, a $C_{1-6}$ acyl group, a carboxy group, a $C_{1-6}$ alkoxy group, a $C_{1-6}$ alkyl group or a phenyl group, or $R^1$ and $R^2$ in combination optionally form a ring together with the adjacent carbon atom (the ring optionally has one or more substituents selected from the group consisting of a halogen atom, a hydroxyl group, an amino group, a $C_{1-6}$ acyl group, a carboxy group, a $C_{1-6}$ alkoxy group, a $C_{1-6}$ alkyl group), or a salt thereof.

2. The compound of claim 1, wherein $R^1$ and $R^2$ are hydrogen atoms, or a salt thereof.

3. A method of producing a compound represented by the following formula (I):

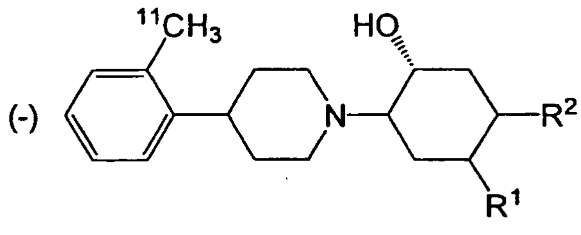

(I)

wherein $R^1$ and $R^2$ are the same or different and each is a hydrogen atom, a halogen atom, a hydroxyl group, an amino group, a $C_{1-6}$ acyl group, a carboxy group, a $C_{1-6}$ alkoxy group, a $C_{1-6}$ alkyl group or a phenyl group, or $R^1$ and $R^2$ in combination optionally form a ring together with the adjacent carbon atom (the ring optionally has one or more substituents selected from the group consisting of a halogen atom, a hydroxyl group, an amino group, a $C_{1-6}$ acyl group, a carboxy group, a $C_{1-6}$ alkoxy group, a $C_{1-6}$ alkyl group),
or a salt thereof, which comprises a step of reacting a compound of the following formula (II):

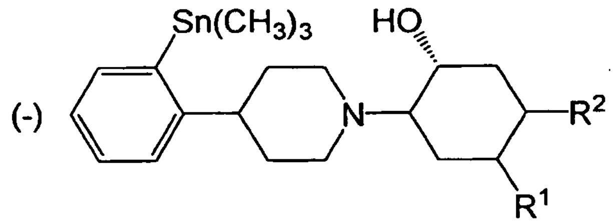

(II)

wherein $R^1$ and $R^2$ are as defined for the aforementioned formula (I), or a salt thereof with $^{11}CH_3I$.

4. The production method of claim 3, wherein $R^1$ and $R^2$ in the aforementioned formula are hydrogen atoms.

5. A diagnostic reagent for a cholinergic neurodegenerative disorder, which comprises a compound of claim 1 or 2, or a salt thereof.

6. The diagnostic reagent of claim 5, wherein the aforementioned cholinergic neurodegenerative disorder is Alzheimer's disease, memory disorder, learning disorder, schizophrenia, cognitive dysfunction, hyperactivity disorder, anxiety neurosis, depression, analgia or Parkinson's disease.

7. A compound represented by the following formula (II):

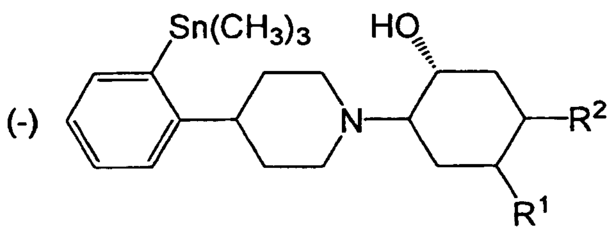

(II)

wherein $R^1$ and $R^2$ are the same or different and each is a hydrogen atom, a halogen atom, a hydroxyl group, an amino group, a $C_{1-6}$ acyl group, a carboxy group, a $C_{1-6}$ alkoxy group, a $C_{1-6}$ alkyl group or a phenyl group, or $R^1$ and $R^2$ in combination optionally form a ring together with the adjacent carbon atom (the ring optionally has one or more substituents selected from the group consisting of a halogen atom, a hydroxyl group, an amino group, a $C_{1-6}$ acyl group, a carboxy group, a $C_{1-6}$ alkoxy group, a $C_{1-6}$ alkyl group), or a salt thereof.

8. The compound of claim 7 or a salt thereof, wherein $R^1$ and $R^2$ in the aforementioned formula are hydrogen atoms.

9. A compound represented by the following formula (Ia):

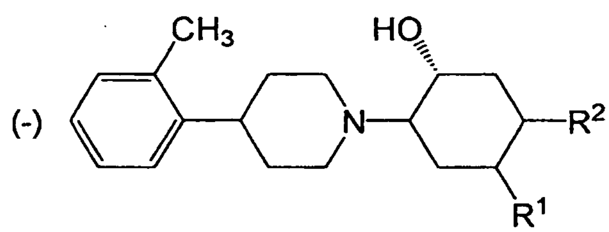

(Ia)

wherein $R^1$ and $R^2$ are the same or different and each is a hydrogen atom, a halogen atom, a hydroxyl group, an amino group, a $C_{1-6}$ acyl group, a carboxy group, a $C_{1-6}$ alkoxy group, a $C_{1-6}$ alkyl group or a phenyl group, or $R^1$ and $R^2$ in combination optionally form a ring together with the adjacent carbon atom (the ring optionally has one or more substituents selected from the group consisting of a halogen atom, a hydroxyl group, an amino group, a $C_{1-6}$ acyl group, a carboxy group, a $C_{1-6}$ alkoxy group, a $C_{1-6}$ alkyl group), or a salt thereof.

10. The compound of claim 9, wherein $R^1$ and $R^2$ in the aforementioned formula are hydrogen atoms, or a salt thereof.

**Patentansprüche**

1. Verbindung, die durch die folgende Formel (I)

(-) 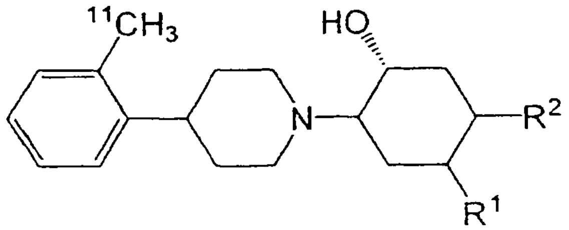 (I)

dargestellt wird, wobei $R^1$ und $R^2$ gleich oder verschieden sind und jeweils ein Wasserstoffatom, ein Halogenatom, eine Hydroxygruppe, eine Aminogruppe, eine $C_{1-6}$-Acylgruppe, eine Carboxygruppe, eine $C_{1-6}$-Alkoxygruppe, eine $C_{1-6}$-Alkylgruppe oder eine Phenylgruppe sind oder $R^1$ und $R^2$ gegebenenfalls in Kombination zusammen mit dem benachbarten Kohlenstoffatom einen Ring bilden (wobei der Ring gegebenenfalls einen oder mehrere Substituenten aufweist, die aus der Gruppe ausgewählt sind, die aus einem Halogenatom, einer Hydroxygruppe, einer Aminogruppe, einer $C_{1-6}$-Acylgruppe, einer Carboxygruppe, einer $C_{1-6}$-Alkoxygruppe und einer $C_{1-6}$-Alkylgruppe besteht); oder ein Salz davon.

2. Verbindung gemäß Anspruch 1, wobei $R^1$ und $R^2$ Wasserstoffatome sind, oder ein Salz davon.

3. Verfahren zur Herstellung einer Verbindung, die durch die folgende Formel (I)

(-) 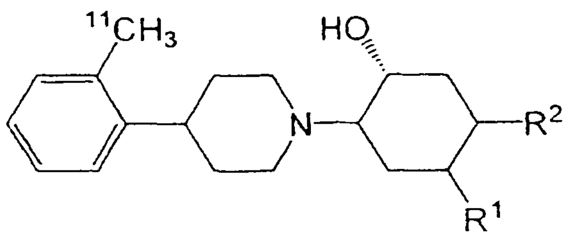 (I)

dargestellt wird, wobei $R^1$ und $R^2$ gleich oder verschieden sind und jeweils ein Wasserstoffatom, ein Halogenatom, eine Hydroxygruppe, eine Aminogruppe, eine $C_{1-6}$-Acylgruppe, eine Carboxygruppe, eine $C_{1-6}$-Alkoxygruppe, eine $C_{1-6}$-Alkylgruppe oder eine Phenylgruppe sind oder $R^1$ und $R^2$ gegebenenfalls in Kombination zusammen mit dem benachbarten Kohlenstoffatom einen Ring bilden (wobei der Ring gegebenenfalls einen oder mehrere Substituenten aufweist, die aus der Gruppe ausgewählt sind, die aus einem Halogenatom, einer Hydroxygruppe, einer Aminogruppe, einer $C_{1-6}$-Acylgruppe, einer Carboxygruppe, einer $C_{1-6}$-Alkoxygruppe und einer $C_{1-6}$-Alkylgruppe besteht); oder eines Salzes davon, umfassend einen Schritt des Umsetzens einer Verbindung, die durch die folgende Formel (II)

(-) 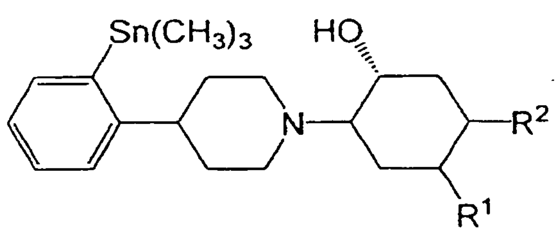 (II)

dargestellt wird, wobei $R^1$ und $R^2$ wie für die oben genannte Formel (I) definiert sind, oder eines Salzes davon mit $^{11}CH_3I$.

4. Herstellungsverfahren gemäß Anspruch 3, wobei $R^1$ und $R^2$ in der oben genannten Formel Wasserstoffatome sind.

5. Diagnostisches Reagens für eine Störung mit Degeneration cholinerger Neuronen, das eine Verbindung gemäß Anspruch 1 oder 2 öder ein Salz davon umfasst.

6. Diagnostisches Reagens gemäß Anspruch 5, wobei es sich bei der oben genannten Störung mit Degeneration cholinerger Neuronen um Alzheimer-Krankheit, Gedächtnisstörungen, Lernstörungen, Schizophrenie, kognitive Dysfunktion, Hyperaktivitätsstörung, Angstneurose, Depression, Analgesie oder Parkinson-Krankheit handelt.

7. Verbindung, die durch die folgende Formel (II)

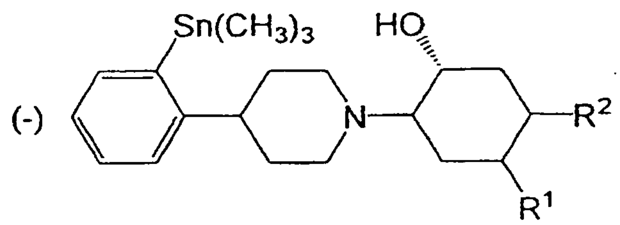

(II)

dargestellt wird, wobei $R^1$ und $R^2$ gleich oder verschieden sind und jeweils ein Wasserstoffatom, ein Halogenatom, eine Hydroxygruppe, eine Aminogruppe, eine $C_{1-6}$-Acylgruppe, eine Carboxygruppe, eine $C_{1-6}$-Alkoxygruppe, eine $C_{1-6}$-Alkylgruppe oder eine Phenylgruppe sind oder $R^1$ und $R^2$ gegebenenfalls in Kombination zusammen mit dem benachbarten Kohlenstoffatom einen Ring bilden (wobei der Ring gegebenenfalls einen oder mehrere Substituenten aufweist, die aus der Gruppe ausgewählt sind, die aus einem Halogenatom, einer Hydroxygruppe, einer Aminogruppe, einer $C_{1-6}$-Acylgruppe, einer Carboxygruppe, einer $C_{1-6}$-Alkoxygruppe und einer $C_{1-6}$-Alkylgruppe besteht); oder ein Salz davon.

8. Verbindung gemäß Anspruch 7 oder ein Salz davon, wobei $R^1$ und $R^2$ in der oben genannten Formel Wasserstoffatome sind.

9. Verbindung, die durch die folgende Formel (Ia)

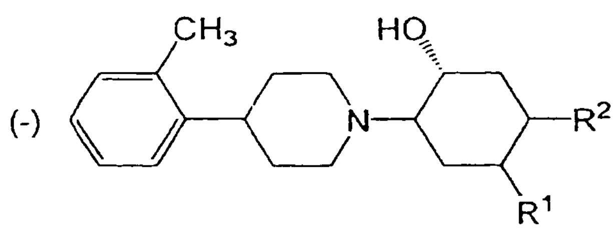

(Ia)

dargestellt wird, wobei $R^1$ und $R^2$ gleich oder verschieden sind und jeweils ein Wasserstoffatom, ein Halogenatom, eine Hydroxygruppe, eine Aminogruppe, eine $C_{1-6}$-Acylgruppe, eine Carboxygruppe, eine $C_{1-6}$-Alkoxygruppe, eine $C_{1-6}$-Alkylgruppe oder eine Phenylgruppe sind oder $R^1$ und $R^2$ gegebenenfalls in Kombination zusammen mit dem benachbarten Kohlenstoffatom einen Ring bilden (wobei der Ring gegebenenfalls einen oder mehrere Substituenten aufweist, die aus der Gruppe ausgewählt sind, die aus einem Halogenatom, einer Hydroxygruppe, einer Aminogruppe, einer $C_{1-6}$-Acylgruppe, einer Carboxygruppe, einer $C_{1-6}$-Alkoxygruppe und einer $C_{1-6}$-Alkylgruppe besteht); oder ein Salz davon.

10. Verbindung gemäß Anspruch 9, wobei $R^1$ und $R^2$ in der oben genannten Formel Wasserstoffatome sind, oder ein Salz davon.

**Revendications**

1. Composé représenté par la formule (I) suivants :

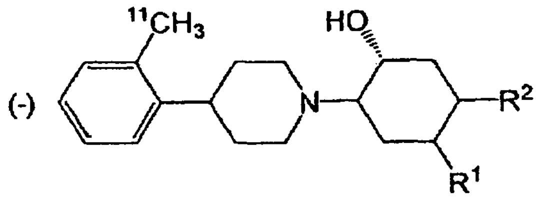

(I)

dans laquelle $R^1$ et $R^2$ sont identiques ou différents et chacun est un atome d'hydrogène, un atome d'halogène, un groupe hydroxyle, un groupe amino, un groupe acyle en $C_{1-6}$, un groupe carboxy, un groupe alcoxy en $C_{1-6}$, un groupe alkyle en $C_{1-6}$ ou un groupe phényle, ou $R^1$ et $R^2$ en combinaison forment éventuellement un cycle avec l'atome de carbone adjacent (le cycle portant éventuellement un ou plusieurs substituants choisis dans le groupe constitué par un atome d'halogène, un groupe hydroxyle, un groupe amino, un groupe acyle en $C_{1-6}$, un groupe carboxy, un groupe alcoxy en $C_{1-6}$, un groupe alkyle en $C_{1-6}$),
ou un sel de celui-ci.

2. Composé selon la revendication 1, dans lequel $R^1$ et $R^2$ sont des atomes d'hydrogène, ou un sel de celui-ci.

3. Procédé de production d'un composé représenté par la formule (I) suivante :

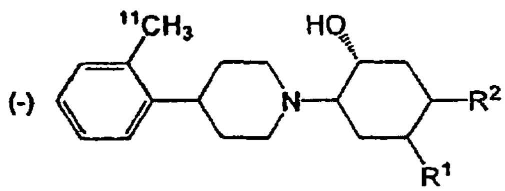

(I)

dans laquelle $R^1$ et $R^2$ sont identiques ou différents et chacun est un atome d'hydrogène, un atome d'halogène, un groupe hydroxyle, un groupe amino, un groupe acyle en $C_{1-6}$, un groupe carboxy, un groupe alcoxy en $C_{1-6}$, un groupe alkyle en $C_{1-6}$ ou un groupe phényle, ou $R^1$ et $R^2$ en combinaison forment éventuellement un cycle avec l'atome de carbone adjacent (le cycle portant éventuellement un ou plusieurs substituants choisis dans le groupe constitué par un atome d'halogène, un groupe hydroxyle, un groupe amino, un groupe acyle en $C_{1-6}$, un groupe carboxy, un groupe alcoxy en $C_{1-6}$, un groupe alkyle en $C_{1-6}$),
ou un sel de celui-ci, qui comprend une étape de réaction d'un composé représenté par la formule (II) suivante :

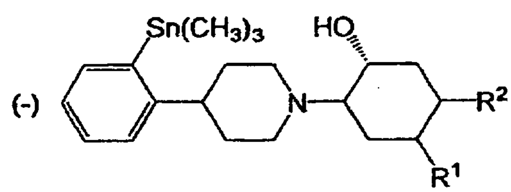

(II)

dans laquelle $R^1$ et $R^2$ sont tels que définis pour la formule (I) susmentionnée,
ou d'un sel de celui-ci, avec $^{11}CH_3I$.

4. Procédé de production selon la revendication 3, dans lequel $R^1$ et $R^2$ dans la formule susmentionnée sont des atomes d'hydrogène.

5. Réactif de diagnostic pour trouble neurodégénératif cholinergique, qui comprend un composé selon la revendication 1 ou 2, ou un sel de celui-ci.

**6.** Réactif de diagnostic selon la revendication 5, dans lequel le trouble neurodégénératif cholinergique susmentionné est la maladie d'Alzheimer, un trouble de la mémoire, un trouble de l'apprentissage, la schizophrénie, un trouble cognitif, un trouble d'hyperactivité, la névrose d'angoisse, la dépression, l'analgie ou la maladie de Parkinson.

**7.** Composé représenté par la formule (II) suivante :

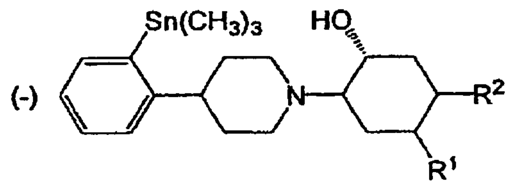

(II)

dans laquelle $R^1$ et $R^2$ sont identiques ou différents et chacun est un atome d'hydrogène, un atome d'halogène, un groupe hydroxyle, un groupe amino, un groupe acyle en $C_{1-6}$, un groupe carboxy, un groupe alcoxy en $C_{1-6}$, un groupe alkyle en $C_{1-6}$ ou un groupe phényle, ou $R^1$ et $R^2$ en combinaison forment éventuellement un cycle avec l'atome de carbone adjacent (le cycle portant éventuellement un ou plusieurs substituants choisis dans le groupe constitué par un atome d'halogène, un groupe hydroxyle, un groupe amino, un groupe acyle en $C_{1-6}$, un groupe carboxy, un groupe alcoxy en $C_{1-6}$, un groupe alkyle en $C_{1-6}$),
ou un sel de celui-ci.

**8.** Composé selon la revendication 7 ou un sel de celui-ci, dans lequel $R^1$ et $R^2$ dans la formule susmentionnée sont des atomes d'hydrogène.

**9.** Composé représenté par la formule (Ia) suivante :

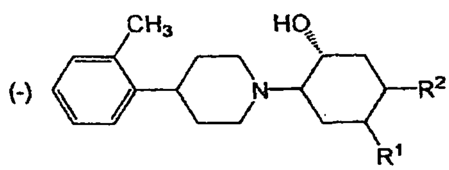

(Ia)

dans laquelle $R^1$ et $R^2$ sont identiques ou différents et chacun est un atome d'hydrogène, un atome d'halogène, un groupe hydroxyle, un groupe amino, un groupe acyle en $C_{1-6}$, un groupe carboxy, un groupe alcoxy en $C_{1-6}$, un groupe alkyle en $C_{1-6}$ ou un groupe phényle, ou $R^1$ et $R^2$ en combinaison forment éventuellement un cycle avec l'atome de carbone adjacent (le cycle portant éventuellement un ou plusieurs substituants choisis dans le groupe constitué par un atome d'halogène, un groupe hydroxyle, un groupe amino, un groupe acyle en $C_{1-6}$, un groupe carboxy, un groupe alcoxy en $C_{1-6}$, un groupe alkyle en $C_{1-6}$),
ou un sel de celui-ci.

**10.** Composé selon la revendication 9, dans lequel $R^1$ et $R^2$ dans la formule susmentionnée sont des atomes d'hydrogène, ou un sel de celui-ci.

FIG. 1

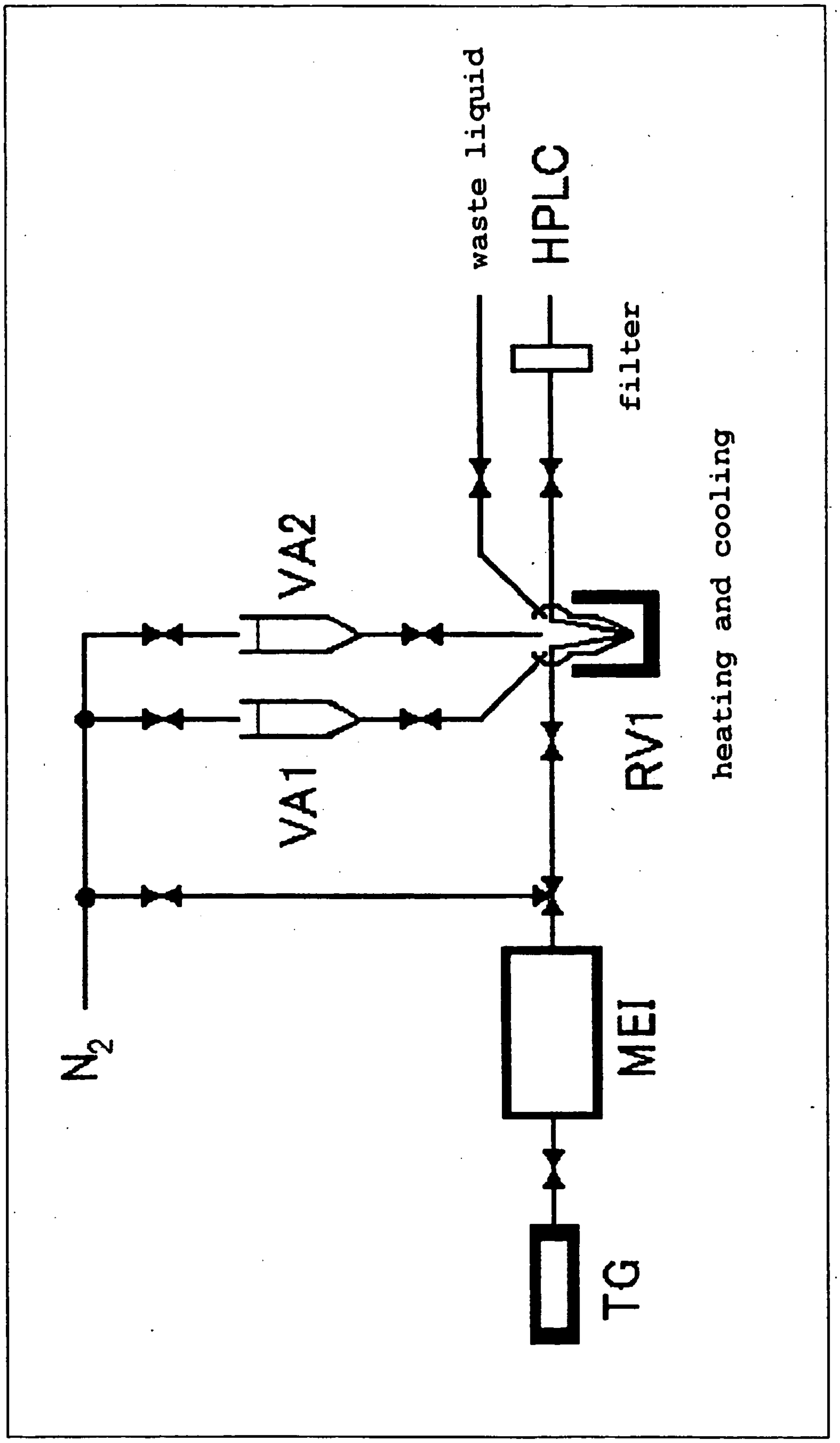
waste liquid
HPLC
filter
VA2
VA1
RV1
heating and cooling
$N_2$
MEI
TG

FIG. 2

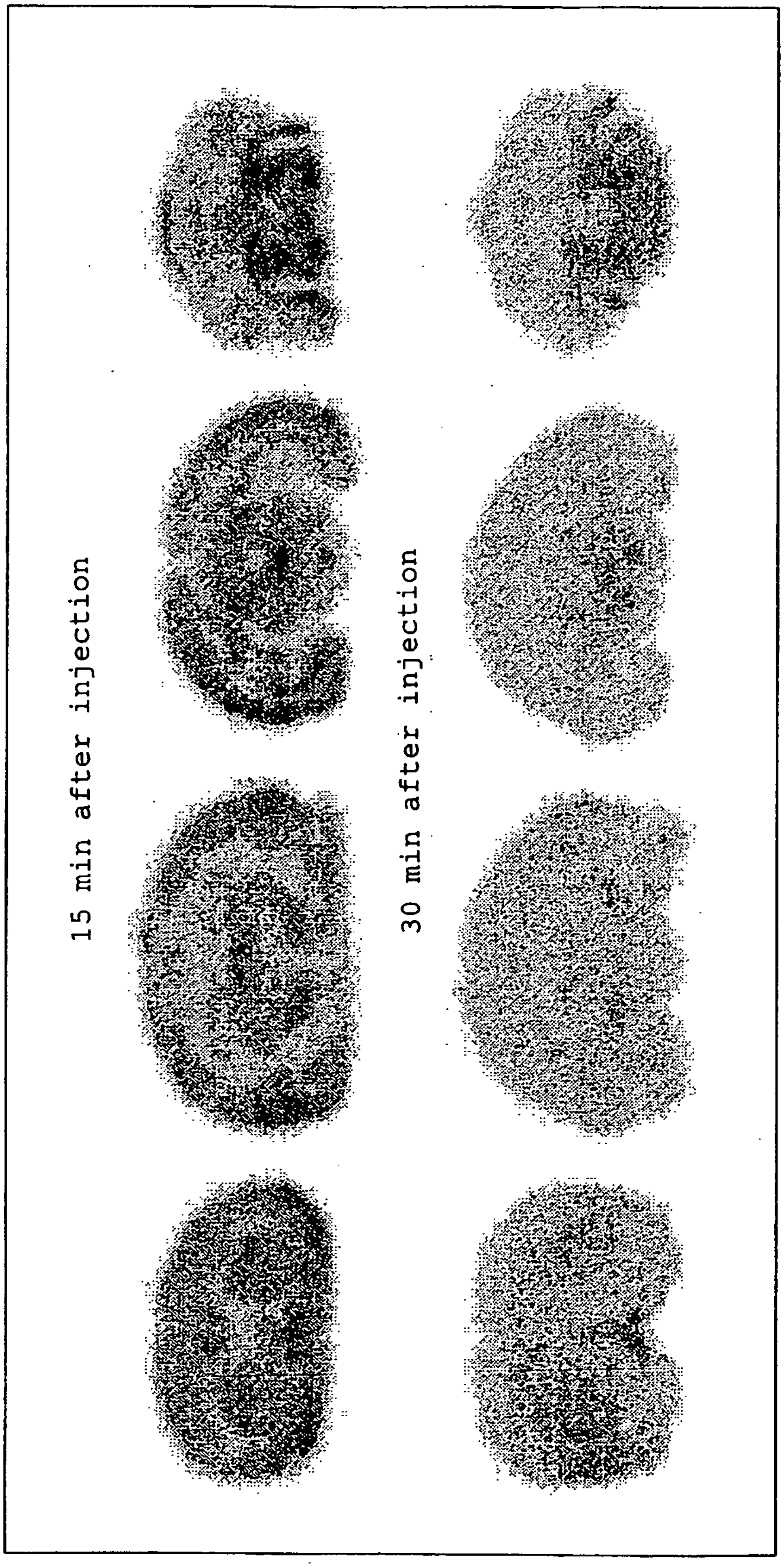

FIG. 3

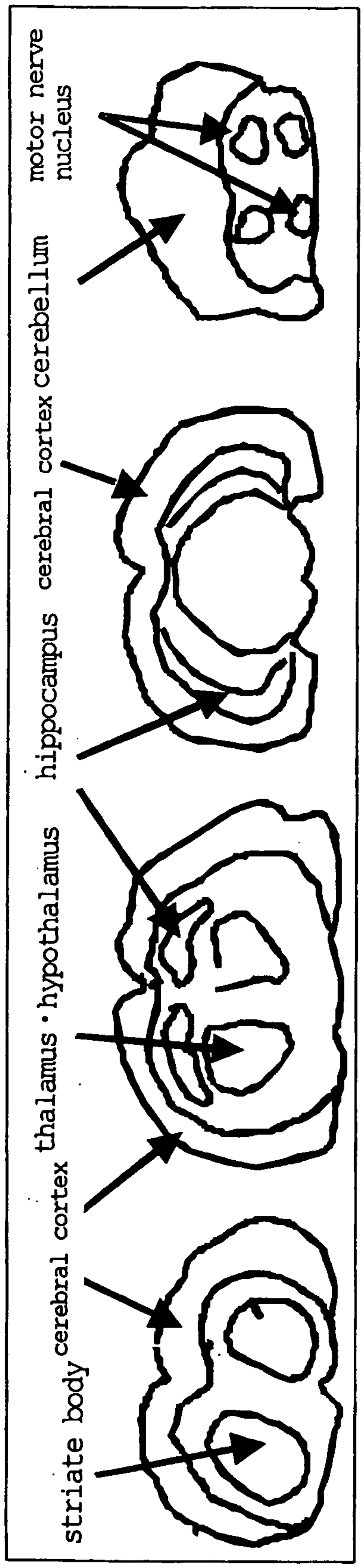
striate body
cerebral cortex
cerebral cortex
thalamus · hypothalamus
hippocampus
cerebral cortex
cerebellum
motor nerve nucleus

FIG. 4

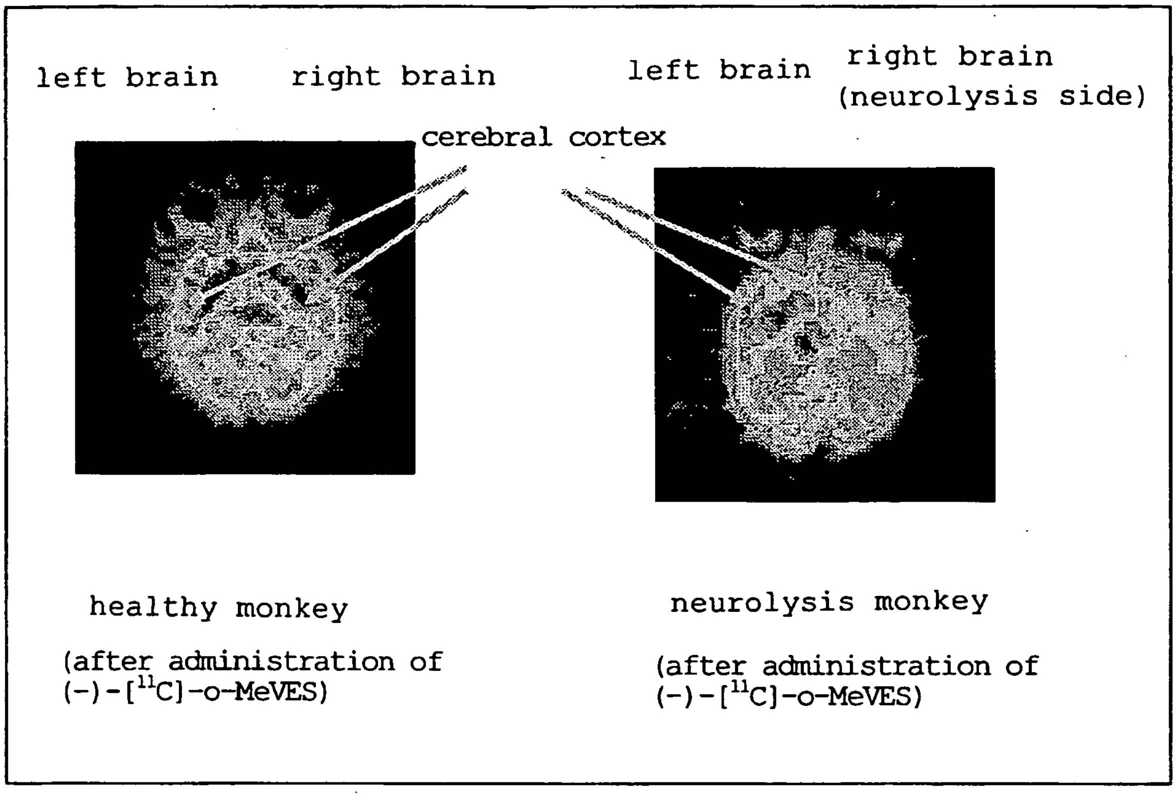
left brain
right brain
left brain
right brain
(neurolysis side)
cerebral cortex
healthy monkey
(after administration of
(-)-[$^{11}$C]-o-MeVES)
neurolysis monkey
(after administration of
(-)-[$^{11}$C]-o-MeVES)

# FIG. 5

left brain right brain left brain right brain (neurolysis side)

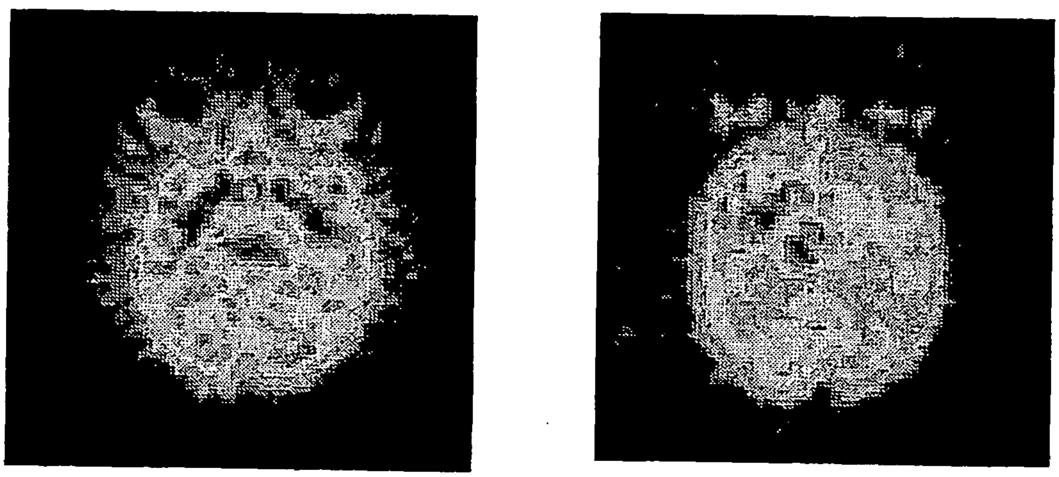

healthy monkey (after administration of (-)-[$^{11}$C]-o-MeVES)

neurolysis monkey (after administration of (-)-[$^{11}$C]-o-MeVES)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005106824 A


### Non-patent literature cited in the description


- **Shiba et al.** *Life Sciences,* 2002, vol. 71, 1591-1598
- **Shiba et al.** Annals of Nuclear Medicine. 2003, vol. 17, 451-456
- *Life Sciences,* 2002, vol. 71, 1591-1598